# EUROPEAN PATENT APPLICATION

(11) **EP 4 817 431 A2**
(43) Date of publication of application: **07.10.2026**
(21) Application number: 26193619.9
(22) Date of filing: 15.11.2023
(51) Int. Cl.: A23L 33/00

(54) **HUMAN MILK OLIGOSACCHARIDES FOR IMPROVING GUT MICROBIOME AND METHODS FOR DETERMINING GUT MATURATION STATUS**

(30) Priority: 15.11.2022 EP 22207543
(62) Document number(s) of the earlier application(s) in accordance with Art. 76 EPC: 23806261.6 / 4 619 002
(71) Applicant: Société des Produits Nestlé S.A., 1800 Vevey (CH)
(72) Inventor: DOGRA, Shaillay, Kumar, 1066 Epalinges (CH)
(74) Representative: Ng, Yen Kar

(57) **Abstract**

The present invention provides methods for inducing a formula-fed infant's gut microbiome trajectory to converge with that of a reference gut microbiome trajectory obtained from human milk-fed infants, the methods comprising administering an effective amount of a mixture of human milk oligosaccharides (HMOs) to the formula-fed infant. The present invention also provides methods for determining the gut maturation status of a formula-fed infant, the methods comprising providing a reference gut microbiome trajectory obtained from human milk-fed infants.

## Description

### FIELD OF THE INVENTION

The present invention relates to methods for inducing an infant's gut microbiome trajectory to converge with that of a reference gut microbiome trajectory and remain or stay convergent with the reference gut microbiome trajectory. The present invention also relates to methods for determining the gut maturation status of an infant and/or toddler.

### BACKGROUND TO THE INVENTION

Breast milk generally provides the reference nutrition for all infants, and exclusive breastfeeding for the first six months is typically recommended. The feeding mode, exclusive or partial breastfeeding versus no breastfeeding during early infancy, has well-recognized effects on the gut microbiome composition and function. For example, a longer duration of exclusive breastfeeding is associated with reduced diarrhoea-related gut microbiota dysbiosis. Furthermore, differences in gut microbiota between breastfed and non-breastfed infants may persist after 6 months of age (see e.g. Ho, N.T., et al., 2018. Nature communications, 9(1), pp.1-13).

Health benefits observed for breastfed infants may include protection against infections, as well as possible reductions in becoming overweight and diabetes development later in life. This indicates that a nutritionally modified early life microbiome maturation can impact later health. The composition of consumed infant formula has been shown in numerous observational cohort studies and randomized controlled trials to have a considerable impact on the microbiome (see e.g. Dogra, S.K., et al., 2021. Microorganisms, 9(10), p.2110).

For example, the addition of two specific human milk oligosaccharides, 2'-fucosyllactose (2'FL) and lacto-N-neotetraose (LNnT), in starter infant formula has been shown to result in a gut microbiome composition closer to that of breastfed infants. However, at 12 months the gut microbiome composition of the breastfed group still significantly differed from the formula groups (see e.g. Berger, B., et al., 2020. Mbio, 11(2), pp.e03196-19).

### SUMMARY OF THE INVENTION

The present inventors have shown that the addition of a mixture of human milk oligosaccharides (HMOs) in starter infant formula, a follow-up formula and growing-up milk can induce a formula-fed infant's gut microbiome trajectory to converge and remain or stay convergent with that of a reference gut microbiome trajectory obtained from human milk-fed group. The gut microbiome trajectories converged earlier and the number of formula-fed infants and toddlers which were outliers was significantly reduced when a mixture of HMOs was added to the starter infant formula, the follow-up formula and growing up milk.

In one aspect, the present invention provides a mixture of human milk oligosaccharides (HMOs) for use in inducing an infant's gut microbiome trajectory to converge with that of a reference gut microbiome trajectory and remain or stay convergent with the reference gut microbiome trajectory.

In another aspect, the present invention provides a method for inducing an infant's gut microbiome trajectory to converge with that of a reference gut microbiome trajectory and remain or stay convergent with the reference gut microbiome trajectory, the method comprising administering an effective amount of a mixture of human milk oligosaccharides (HMOs) to the infant.

In another aspect, the present invention provides use of a mixture of human milk oligosaccharides (HMOs) to induce an infant's gut microbiome trajectory to converge with that of a reference gut microbiome trajectory and remain or stay convergent with the reference gut microbiome trajectory.

The mixture of HMOs may comprise any suitable HMOs, which may be administered in any suitable form, in any suitable amounts, and for any suitable duration. Suitably, the mixture of HMOs comprises at least one fucosylated oligosaccharide, at least one N-acetylated oligosaccharide, and at least one sialylated oligosaccharide. Suitably, the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL). Suitably, the mixture of HMOs is administered in the form of an infant formula and/or growing-up milk. Suitably, the mixture of HMOs comprises or consists of: (i) 2'FL in an amount of from about 55 wt% to about 60 wt%; (ii) diFL in an amount of from about 5 wt% to about 7 wt%; (iii) LNT in an amount of from about 18 wt% to about 20 wt%; (iv) 3'-SL in an amount of from about 6 wt% to about 8 wt%; and (v) 6'-SL in an amount of from about 9 wt% to about 11 wt%, based on the total weight of HMOs. Suitably, the mixture of HMOs is administered to the infant until at least about 6 months of age, until at least about 9 months of age, until at least about 12 months of age, or until at least about 15 months of age.

In preferred embodiments, the mixture of HMOs is administered in the form of a starter infant formula, a follow-up formula, and/or growing-up milk. Suitably, the mixture of HMOs is administered in the form of a starter infant formula comprising a total amount of HMOs of from about 0.5 g/L to about 5.0 g/L, from about 1.0 g/L to about 3.0 g/L, or from about 1.5 g/L to about 2.5 g/L; a follow-up formula comprising a total amount of HMOs of from about 0.1 g/L to about 2.0 g/L, from about 0.2 g/L to about 1.0 g/L, from about 0.3 g/L to about 0.8 g/L, from about 0.35 g/L to about 0.65 g/L; and/or a growing-up milk comprising a total amount of HMOs of from about 0.1 g/L to about 1.0 g/L, from about 0.2 g/L to about 0.8 g/L, from about 0.28 g/L to about 0.52 g/L, or from about 0.3 g/L to about 0.5 g/L.

Suitably, the mixture of HMOs is administered in the form of a starter infant formula comprising: (i) 2'FL in an amount of from about 0.5 g/L to about 3.0 g/L; (ii) diFL in an amount of from about 0.05 g/L to about 0.3 g/L; (iii) LNT in an amount of from about 0.1 g/L to about 1.0 g/L; (iv) 3'-SL in an amount of from about 0.05 g/L to about 0.3 g/L; and (v) 6'-SL in an amount of from about 0.05 g/L to about 0.5 g/L. In some embodiments, the mixture of HMOs is administered in the form of a starter infant formula comprising: (i) 2'FL in an amount of from about 0.70 g/L to about 1.05 g/L or from about 1.16 g/L to about 1.74 g/L; (ii) diFL in an amount of from about 0.05 g/L to about 0.11 g/L or from about 0.12 g/L to about 0.18 g/L; (iii) LNT in an amount of from about 0.23 g/L to about 0.36 g/L or from about 0.39 g/L to about 0.58 g/L; (iv) 3'-SL in an amount of from about 0.09 g/L to about 0.13 g/L or from about 0.14 g/L to about 0.21 g/L; and (v) 6'-SL in an amount of from about 0.12 g/L to about 0.17 g/L or from about 0.19 g/L to about 0.28 g/L. Suitably, the mixture of HMOs is administered in the form of a follow-up formula comprising: (i) 2'FL in an amount of from about 0.19 g/L to about 0.34 g/L; (ii) diFL in an amount of from about 0.03 g/L to about 0.05 g/L; (iii) LNT in an amount of from about 0.06 g/L to about 0.11 g/L; (iv) 3'-SL in an amount of from about 0.04 g/L to about 0.09 g/L; and (v) 6'-SL in an amount of from about 0.03 g/L to about 0.06 g/L. Suitably, the mixture of HMOs is administered in the form of a growing-up milk comprising: (i) 2'FL in an amount of from about 0.15 g/L to about 0.28 g/L; (ii) diFL in an amount of from about 0.01 g/L to about 0.04 g/L; (iii) LNT in an amount of from about 0.05 g/L to about 0.09 g/L; (iv) 3'-SL in an amount of from about 0.03 g/L to about 0.08 g/L; and (v) 6'-SL in an amount of from about 0.03 g/L to about 0.05 g/L.

In some embodiments, the mixture of HMOs is administered in the form of a starter infant formula comprising a total amount of HMOs of about 1.5 g/L. In some embodiments, the mixture of HMOs is administered in the form of a starter infant formula comprising: (i) 2'FL in an amount of about 0.87 g/L; (ii) diFL in an amount of about 0.10 g/L; (iii) LNT in an amount of about 0.29 g/L; (iv) 3'-SL in an amount of about 0.11 g/L; and (v) 6'-SL in an amount of about 0.14 g/L. In some embodiments, the mixture of HMOs is administered in the form of a starter infant formula comprising a total amount of HMOs of about 2.5 g/L. In some embodiments, the mixture of HMOs is administered in the form of a starter infant formula comprising: (i) 2'FL in an amount of about 1.45 g/L; (ii) diFL in an amount of about 0.14 g/L; (iii) LNT in an amount of about 0.48 g/L; (iv) 3'-SL in an amount of about 0.18 g/L; and (v) 6'-SL in an amount of about 0.24 g/L. In some embodiments, the mixture of HMOs is administered in the form of a follow-up formula comprising a total amount of HMOs of about 0.5 g/L. In some embodiments, the mixture of HMOs is administered in the form of a follow-up formula comprising: (i) 2'FL in an amount of about 0.26 g/L; (ii) diFL in an amount of about 0.04 g/L; (iii) LNT in an amount of about 0.09 g/L; (iv) 3'-SL in an amount of about 0.06 g/L; and (v) 6'-SL in an amount of about 0.05 g/L. In some embodiments, the mixture of HMOs is administered in the form of a growing-up milk comprising a total amount of HMOs of about 0.4 g/L. In some embodiments, the mixture of HMOs is administered in the form of a growing-up milk comprising: (i) 2'FL in an amount of about 0.21 g/L; (ii) diFL in an amount of about 0.03 g/L; (iii) LNT in an amount of about 0.07 g/L; (iv) 3'-SL in an amount of about 0.06 g/L; and (v) 6'-SL in an amount of about 0.04 g/L.

The starter infant formula, follow-up formula, and growing-up milk may each comprise any other suitable components. Suitably, the starter infant formula, follow-up formula, and growing-up milk each comprise from about 60 kcal/100mL to about 80 kcal/100mL, protein in an amount of from about 1.5 g/100kcal to about 2.5 g/100kcal, carbohydrate in an amount of from about 8 g/100kcal to about 15 g/100kcal, and lipids in an amount of from about 3 g/100kcal to about 8 g/100kcal.

The infant's gut microbiome trajectory may converge with that of the reference gut microbiome trajectory and remain or stay convergent with the reference gut microbiome trajectory at about 12 months of age or earlier, about 11 months of age or earlier, about 10 months of age or earlier, or about 9 months of age or earlier. The infant's gut microbiome trajectory may converge with that of the reference gut microbiome trajectory and remain or stay convergent with the reference gut microbiome trajectory at about 6 months of age or later, at about 7 months of age or later, at about 8 months of age or later, or at about 9 months of age or later. The infant's gut microbiome trajectory may converge with that of the reference gut microbiome trajectory and remain or stay convergent with the reference gut microbiome trajectory at about 6 to about 12 months of age, at about 7 to about 11 months of age, at about 8 to 10 months of age, or at about 9 months of age.

The infant's gut microbiome trajectory and reference gut microbiome trajectory may be obtained by any suitable method. The reference gut microbiome trajectory may be obtained from human milk-fed group (infants and/or toddlers). Suitably, the gut microbiome trajectory is a gut microbiome age trajectory or a gut microbiome diversity trajectory. Suitably, the gut microbiome trajectory is a gut microbiome age trajectory. Suitably, the gut microbiome age trajectory is obtained using genus-level data, species-level data, and/or functional data from gut microbiome data.

The infant may be any infant with a gut microbiome composition that differs from the reference gut microbiome trajectory. The infant may be a formula-fed infant. Suitably, the infant is full-term. Suitably, the infant was delivered by caesarean section.

Inducing an infant's gut microbiome trajectory to converge with that of a reference gut microbiome trajectory and remain or stay convergent with the reference gut microbiome trajectory can be associated with health benefits. For example, inducing the infant's gut microbiome trajectory to converge with that of a reference gut microbiome trajectory may promote gut maturation. Inducing the infant's gut microbiome trajectory to converge with that of a reference gut microbiome trajectory may promote maturation of the infant's gut microbiome, gut metabolism, gut barrier function and/or gut immune function.

In one aspect, the present invention provides a method for determining the gut maturation status of a formula-fed infant or toddler, wherein the method comprises:
(a) providing gut microbiome data from a population of human-milk fed group (infants and/or toddlers);
(b) training a regression model on the gut microbiome data; and
(c) providing gut microbiome data from the formula-fed infant or toddler and determining whether the formula-fed infant or toddler is an outlier or not in the trained regression model,
wherein the gut maturation status of the formula-fed infant or toddler is normal if the formula-fed infant or toddler is not an outlier in the trained regression model, and/or wherein the gut maturation status of the formula-fed infant or toddler is not normal if the formula-fed infant or toddler is an outlier in the trained regression model.

In another aspect, the present invention provides a method for determining the gut maturation status of a formula-fed infant or toddler, wherein the method comprises:
(a) providing gut microbiome data from a population of human-milk fed group (infants and/or toddlers);
(b) training a regression model on the gut microbiome data to provide a gut microbiome trajectory; and
(c) providing gut microbiome data from the formula-fed infant or toddler and determining whether the formula-fed infant or toddler is on or off the gut microbiome trajectory,
wherein the gut maturation status of the formula-fed infant or toddler is normal if the formula-fed infant or toddler is on the gut microbiome trajectory, and/or wherein the gut maturation status of the formula-fed infant or toddler is not normal if the formula-fed infant or toddler is off the gut microbiome trajectory.

Any suitable statistical method may be used to determine whether the formula-fed infant or toddler is an outlier in the trained regression model and/or on or off the gut microbiome trajectory. Suitably, the formula-fed infant or toddler is an outlier and/or off trajectory based on the standard errors (SE), confidence intervals, prediction intervals, and/or standard deviations in the trained regression model or gut microbiome trajectory. Suitably, the formula-fed infant or toddler is an outlier and/or off trajectory if their gut microbiome data is -2SE or less or 2SE or more, -2.5SE or less or 2.5SE or more, or -3SE or less or 3SE or more from the trained regression line or gut microbiome trajectory, if their gut microbiome data falls outside the 90%, 95% or 99% confidence interval in the trained regression model or gut microbiome trajectory, if their gut microbiome data falls outside the 90%, 95% or 99% prediction interval in the trained regression model or gut microbiome trajectory, and/or if they have a Z-score of -2 or less or 2 or more, -2.5 or less or 2.5 or more, or -3 or less or 3 or more in the trained regression model or gut microbiome trajectory.

In another aspect, the present invention provides a data processing system comprising means for carrying out a method for determining the gut maturation status of a formula-fed infant or toddler according to the present invention.

In another aspect, the present invention provides a processor configured to perform a method for determining the gut maturation status of a formula-fed infant or toddler according to the present invention.

In another aspect, the present invention provides a computer-readable medium comprising instructions which, when executed by a computer, cause the computer to carry out a method for determining the gut maturation status of a formula-fed infant or toddler according to the present invention.

In another aspect, the present invention provides a computer program comprising instructions which, when the program is executed by a computer, cause the computer to carry out a method for determining the gut maturation status of a formula-fed infant or toddler according to the present invention.

In another aspect, the present invention provides a computer-readable data carrier having stored thereon a computer program according to the present invention.

In another aspect, the present invention provides a data carrier signal carrying a computer program according to the present invention.

In another aspect, the present invention provides use of one or more reference gut microbiome trajectories to determine the gut maturation status of a formula-fed infant or toddler following administration of a mixture of HMOs.

### DESCRIPTION OF DRAWINGS

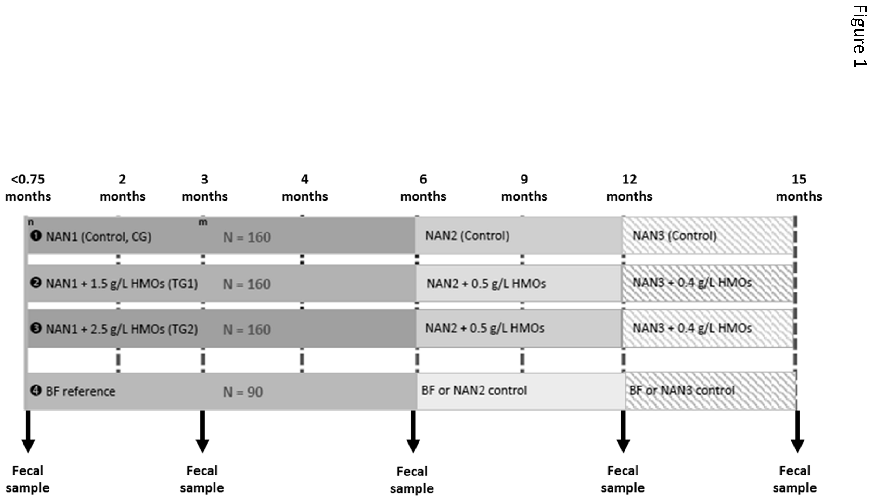

**Figure 1** **- Schematic of HMOs trial**
   Healthy full-term infants were randomly assigned to a standard cow's milk-based starter infant formula (control group, CG); the same formula with 1.5 g/L HMOs (test group 1, TG1); or with 2.5 g/L HMOs (test group 2, TG2); or a human milk-fed group (reference, HMG). Fecal samples were collected at enrolment, 3, 6, 12, and 15 months of age.
**Figure 2** **- Modelling of gut microbiome age**
   The overall process of modelling the microbiome age is illustrated.
**Figure 3** **- Example gut microbiome trajectories by model**
   LOESS fit microbiome age trajectories for each feeding group using an age predictor trained on data from vaginally-delivered HMG group (infants and/or toddlers) using: **(A)** Genus-level data (selected 10 features, R² = 0.862); **(B)** MGS Species-level data (selected 20 features, R² = 0.881); **(C)** MGS Species-level data (selected 25 features, R² = 0.844); **(D)** CAZyme data (selected 30 features, R² = 0.658); **(E)** Shannon index by gene. Shaded areas indicate 95 % confidence intervals.

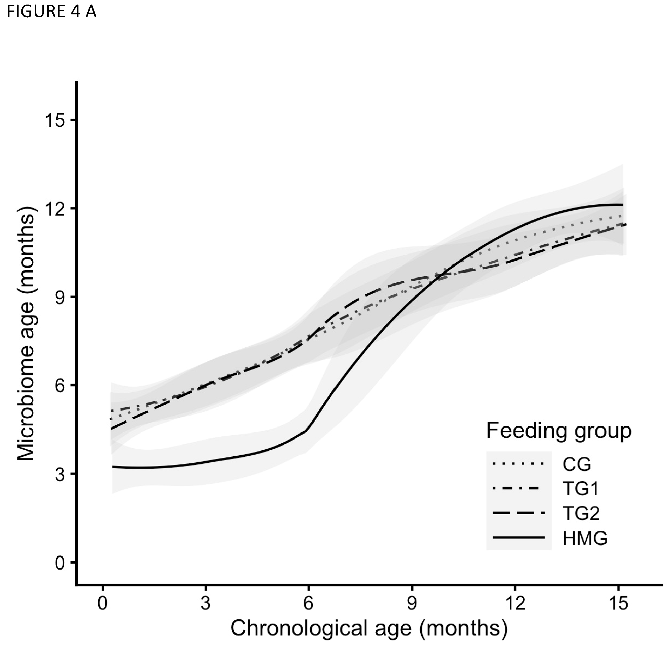

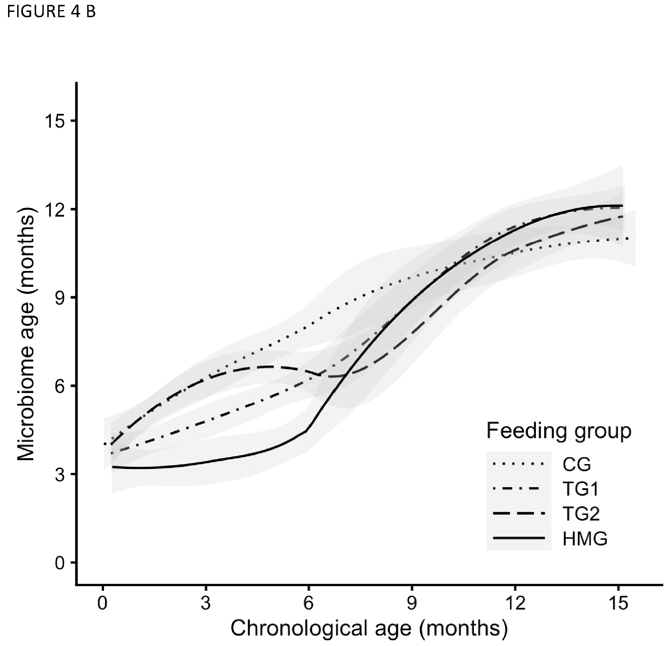

**Figure 4** **- Example gut microbiome trajectories by mode of delivery**
   LOESS fit microbiome age trajectories for each feeding group using an age predictor trained on data from vaginally-delivered HMG group (infants and/or toddlers) using CAZyme data (selected 30 features) for: **(A)** vaginally-delivered formula-fed group (infants and/or toddlers); **(B)** caesarean-delivered formula-fed group (infants and/or toddlers). Shaded areas indicate 95 % confidence intervals.

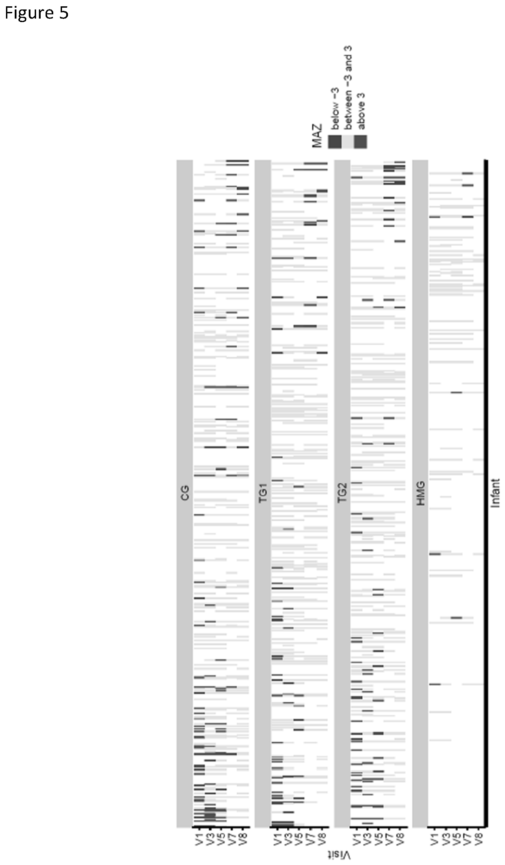

**Figure 5** **- Example outliers in genus-level data**
   MAZ values were calculated from an age predictor trained on data from vaginally-delivered HMG group (infants and/or toddlers) using Genus-level data (selected 10 features, optimized with RMSE, R² = 0.862).

### DETAILED DESCRIPTION

Various preferred features and embodiments of the present invention will now be described by way of non-limiting examples. The skilled person will understand that they can combine all features of the invention disclosed herein without departing from the scope of the invention as disclosed.

It must be noted that as used herein and in the appended claims, the singular forms "a", "an", and "the" include plural referents unless the context clearly dictates otherwise. The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

Numeric ranges are inclusive of the numbers defining the range. As used herein the term "about" means approximately, in the region of, roughly, or around. When the term "about" is used in conjunction with a numerical value or range, it modifies that value or range by extending the boundaries above and below the numerical value(s) set forth. In general, the terms "about" and "approximately" are used herein to modify a numerical value(s) above and below the stated value(s) by 10%.

The concentration of components in the compositions described herein may refer to the concentration after the composition has been reconstituted e.g. with water.

The publications discussed herein are provided solely for their disclosure prior to the filing date of the present application. Nothing herein is to be construed as an admission that such publications constitute prior art to the claims appended hereto.

The methods and systems disclosed herein can be used by doctors, health-care professionals, lab technicians, infant and/or toddler care providers and so on.

### Human milk oligosaccharide (HMO) mixture

The present invention provides a mixture of human milk oligosaccharides (HMOs) for use in inducing an infant's gut microbiome trajectory to converge with that of a reference gut microbiome trajectory and remain or stay convergent with the reference gut microbiome trajectory.

### Human milk oligosaccharides (HMOs)

Any suitable mixture of HMOs may be used in the present invention.

As used herein, "human milk oligosaccharides" or "HMOs" (also known as human milk glycans) are short polymers of simple sugars that can be found in high concentrations in human breast milk. Many different kinds of HMOs are found in human milk. Each individual oligosaccharide is based on a combination of glucose, galactose, sialic acid, fucose and/or N-acetylglucosamine with many and varied linkages between them, thus accounting for the enormous number of different oligosaccharides in human milk. Most HMOs have a lactose moiety at their reducing end, while sialic acid and/or fucose (when present) occupy terminal positions at the non-reducing ends. HMOs can be acidic (e.g. charged sialic acid containing oligosaccharides) or neutral (e.g. fucosylated oligosaccharides).

The mixture of HMOs may comprise two or more individual HMOs, three or more individual HMOs, four or more individual HMOs, or five or more individual HMOs. In some embodiments, the mixture of HMOs comprises five or more individual HMOs. In some embodiments, the mixture of HMOs comprises five individual HMOs.

Suitable HMOs for use in the present invention and which are abundant in human milk may include 2'-fucosyllactose (2'FL), 2',3-difucosyllactose (diFL), 3-fucosyllactose (3FL), lacto-N-fucopentaose-I (LNFP-I), lacto-N-fucopentaose-II (LNFP-II), lacto-N-fucopentaose-III (LNFP-III), lacto-N-difucosylhexaose-I (LNDFH-I), lacto-N-tetraose (LNT), lacto-N-neotetraose (LNnT), 3'-sialyllactose (3'-SL), and 6'-sialyllactose (6'-SL), and disyallacto-N-tetraose (DSLNT).

The HMOs used in the present invention may be obtained by any suitable method. Suitable methods for synthesising HMOs will be well known to those of skill in the art. For example, processes have been developed for producing HMOs by microbial fermentations, enzymatic processes, chemical syntheses, or combinations of these technologies (see e.g. Zeuner et al., 2019. Molecules, 24(11), p.2033).

In some embodiments, the mixture of HMOs comprises at least one fucosylated oligosaccharide, at least one N-acetylated oligosaccharide, and/or at least one sialylated oligosaccharide. In some embodiments, the mixture of HMOs comprises at least one fucosylated oligosaccharide, at least one N-acetylated oligosaccharide, and at least one sialylated oligosaccharide.

In some embodiments, the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL).

In some embodiments, the mixture of HMOs consists of 2'-fucosyllactose (2'FL), 2',3-difucosyllactose (diFL), lacto-N-tetraose (LNT), 3'-sialyllactose (3'-SL), and 6'-sialyllactose (6'-SL).

### Fucosylated oligosaccharides

In some embodiments, the mixture of HMOs comprises at least one fucosylated oligosaccharide.

Suitably, the at least one fucosylated oligosaccharide comprises of consists of 2'-fucosyllactose (2'FL), 2',3-difucosyllactose (diFL), 3-fucosyllactose (3FL), lacto-N-fucopentaose-I (LNFP-I), lacto-N-fucopentaose-II (LNFP-II), lacto-N-fucopentaose-III (LNFP-III), lacto-N-fucopentaose-V (LNFP-V), lacto-neofucopentaose V (LNnFP-V), lacto-N-difucosylhexaose-I (LNDFH-1), lacto-N-neodifucosylhexaose (LNnDFH), monofucosyllacto-n-hexaose-III (MFNLH-III), difucosyllacto-N-hexaose-a (DFLNHa), or any combination thereof.

In some embodiments, the at least one fucosylated oligosaccharide comprises of consists of 2'-fucosyllactose (2'FL) and/or 2',3-difucosyllactose (diFL). In some embodiments, the at least one fucosylated oligosaccharide consists of 2'-fucosyllactose (2'FL) and 2',3-difucosyllactose (diFL).

The at least one fucosylated oligosaccharide may be obtained by any suitable method. For example, 2'FL may be produced by biotechnological means using specific fucosyltransferases and/or fucosidases either through the use of enzyme-based fermentation technology (recombinant or natural enzymes) or microbial fermentation technology. In the latter case, microbes may either express their natural enzymes and substrates or may be engineered to produce respective substrates and enzymes. Alternatively, 2'FL may be produced by chemical synthesis from lactose and free fucose. diFL may be synthesized by enzymatic, biotechnological and/or chemical processes.

### N-acetylated oligosaccharides

In some embodiments, the mixture of HMOs comprises at least one N-acetylated oligosaccharide.

Suitably, the at least one N-acetylated oligosaccharide comprises of consists of lacto-N-tetraose (LNT), lacto-N-neotetraose (LNnT), a N-acetyl-glucosamine, a N-acetylgalactosamine, or any combination thereof.

In some embodiments, the at least one N-acetylated oligosaccharide consists of lacto-N-tetraose (LNT).

The N-acetylated oligosaccharides may be obtained by any suitable method. For example, LNnT may be synthesised chemically by enzymatic transfer of saccharide units from donor moieties to acceptor moieties using glycosyltransferases. Alternatively, LNnT may be prepared by chemical conversion of Keto-hexoses (e.g. fructose) either free or bound to an oligosaccharide (e.g. lactulose) into N-acetylhexosamine or an N-acetylhexosamine-containing oligosaccharide. LNT may be synthesized by enzymatic, biotechnological and/or chemical processes.

### Sialylated oligosaccharide

In some embodiments, the mixture of HMOs comprises at least one sialylated oligosaccharide.

Suitably, the at least one sialylated oligosaccharide comprises of consists of 3'-sialyllactose (3'-SL), 6'-sialyllactose (6'-SL), syalyllacto-N-tetraose b (LSTb), syalyllacto-N-tetraose c (LSTc), disyallacto-N-tetraose (DSLNT), or any combination thereof.

In some embodiments, the at least one sialylated oligosaccharide comprises or consists of 3'-sialyllactose (3'-SL) and/or 6'-sialyllactose (6'-SL). In some embodiments, the at least one sialylated oligosaccharide consists of 3'-sialyllactose (3'-SL) and 6'-sialyllactose (6'-SL).

The sialylated oligosaccharides may be obtained by any suitable method. For example, 3'-sialyllactose (3'-SL) and/or 6'-sialyllactose (6'-SL) may be isolated by chromatographic or filtration technology from a natural source such as animal milks. Alternatively, they may be produced by biotechnological means using specific sialyltransferases or sialidases, neuraminidases, either by an enzyme based fermentation technology (recombinant or natural enzymes), by chemical synthesis or by a microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP), from DP=1 onwards. Alternatively, sialyllactoses may be produced by chemical synthesis from lactose and free sialic acid.

### Form of administration

The mixture of HMOs may be administered in any suitable form. For example, the mixture of HMOs may be administered in the form of a nutritional composition, a medical food product for clinical nutrition, or a supplement.

In some embodiments, the mixture of HMOs is administered in the form of a nutritional composition. As used herein, a "nutritional composition" may refer to a composition which nourishes a subject. A nutritional composition is usually to be taken orally or intravenously, and it usually includes a lipid or fat source and a protein source.

The nutritional composition may be a synthetic nutritional composition. As used herein, a "synthetic nutritional composition" may refer to a mixture obtained by chemical and/or biological means, which can be chemically identical to the mixture naturally occurring in mammalian milks (i.e., the synthetic composition is not breast milk).

The nutritional composition may be any suitable nutritional composition in which the mixture of HMOs can be incorporated, such as a nutritional composition in the form of a food or beverage product, a nutritional supplement, a nutraceutical composition, or a pharmaceutical composition. The nutritional composition may be in solid (e.g. powder), liquid or semi-liquid form. Suitably, the nutritional composition is in a form suitable for feeding infants, such as an infant formula, a milk fortifier, or a supplement. The nutritional composition can also be in a form for young children such as a yoghurt or a medical food.

In preferred embodiments, the mixture of HMOs is administered in the form of an infant formula. An infant formula can be a starter infant formula, a preterm infant formula, a milk fortifier, a follow-up formula, a baby-food formula, an infant cereal formula, or a growing-up milk. As used herein, the term "infant formula" may refer to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-up formulae). It may also refer to a nutritional composition intended for infants and as defined in Codex Alimentarius (Codex STAN 72-1981) and Infant Specialities (incl. Food for Special Medical Purpose).

In some embodiments, the mixture of HMOs is administered in the form of a starter infant formula. Generally a "starter infant formula" is intended for infants from birth as breast-milk substitute.

In some embodiments, the mixture of HMOs is administered in the form of follow-up formula. A "follow-up formula" or "follow-on formula" may be given from the sixth month onwards. It may constitute the principal liquid element in the progressively diversified diet of this category of person.

In some embodiments, the mixture of HMOs is administered in the form of a growing-up milk. The term "growing-up milk" (or GUM) as used herein may refer to a milk formula product given from one year onwards. It is generally a diary-based beverage adapted for the specific nutritional needs of young children.

In some embodiments, the mixture of HMOs is administered in the form of a preterm infant formula. The term "preterm infant formula" as used herein may refer to an infant formula intended for a preterm infant.

In some embodiments, the mixture of HMOs is administered in the form of a milk fortifier. The term "milk fortifier" as used herein may refer to liquid or solid nutritional compositions suitable for mixing with infant formula.

In some embodiments, the mixture of HMOs is administered in the form of a baby-food formula. The term "baby food formula" as used herein may refer to a foodstuff intended for particular nutritional use by infants or children such as young children, during the first years of life.

In some embodiments, the mixture of HMOs is administered in the form of an infant cereal composition. The term "infant cereal composition" as used herein may refer to a foodstuff intended for particular nutritional use by infants or children such as young children, during the first years of life.

In some embodiments, the mixture of HMOs is administered at least in the form of a starter infant formula, a follow-up formula, and/or a growing-up milk. In some embodiments, the mixture of HMOs is administered at least in the form of a starter infant formula. In some embodiments, the mixture of HMOs is administered at least in the form of a starter infant formula and a follow-up formula. In some embodiments, the mixture of HMOs is administered at least in the form of a starter infant formula, a follow-up formula, and a growing-up milk.

In some embodiments, the mixture of HMOs is administered in the form of a starter infant formula. In some embodiments, the mixture of HMOs is administered in the form of a starter infant formula and a follow-up formula. In some embodiments, the mixture of HMOs is administered in the form of a starter infant formula, a follow-up formula, and a growing-up milk.

In other embodiments, the mixture of HMOs is administered in the form of a fortifier. The fortifier can be a formula fortifier such as an infant formula fortifier. The fortifier may be a particularly advantageous embodiment when the infant or young child is born preterm.

In other embodiments, the mixture of HMOs is administered in the form of a supplement. As used herein, a "supplement" or "dietary supplement" may be used to complement the nutrition of a subject (it is typically used as such but it might also be added to any kind of compositions intended to be ingested by the subject).

When the composition is a supplement, it can be provided in the form of unit doses. Supplements are typically present in the form of a liquid, a gel, a powder, a tablet, or a capsule. Powder supplements typically encompass supplements to be dissolved in water or to be sprinkled on food or in a beverage. Such supplements are intended to provide additional nutrients and/or a health benefit to the subject consuming it. A supplement can be used for providing nutrients and/or a health benefit to human beings, as well as to animals, as defined above. Supplements include for example powder supplements to be added to breast milk, for example for premature or low birth weight infants.

In other embodiments, the mixture of HMOs is administered in the form of a pharmaceutical product. Pharmaceutical products include for example drops, syrups, powder, tablet or capsule products intended to treat or prevent an adverse medical condition in a subject in need thereof. In other embodiments, the mixture of HMOs is administered in the form of a nutraceutical product.

### Other components

In addition to the mixture of HMOs, a nutritional composition of the invention, and especially the infant formula, generally contains a protein source, a carbohydrate source and a lipid source.

A nutritional composition according to the invention, and especially an infant formula of the invention, may contain a protein source. The protein may be present in an amount of from about 1.5 to about 3.0 g/100kcal, from about 1.5 to about 2.5 g/100kcal, from about 1.6 to about 2.5 g/100kcal, or from about 1.6 to about 2.25 g/100kcal. In some embodiments, the protein amount is present in an amount of about 2.0 g g/100kcal or less, e.g. from about 1.8 to about 2.0 g/100kcal, or about 1.9 g/100kcal.

Protein sources based on, for example, whey, casein and mixtures thereof may be used as well as plant based protein sources, for example, based on soy. As far as whey proteins are concerned, the protein source may be based on acid whey or sweet whey or mixtures thereof and may include alpha-lactalbumin and beta-lactoglobulin in any desired proportions. In some embodiments, the protein source is whey predominant (i.e. more than 50% of proteins are coming from whey proteins, such as 60% or more or 70% or more).

The proteins may be intact or hydrolysed or a mixture of intact and hydrolysed proteins.

The term "intact" in the context of the present invention may mean that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" in the context of the present invention may mean proteins which have been hydrolysed or broken down into its component amino acids. The proteins may be either fully or partially hydrolysed. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10%> by weight of lysine; for example about 7% by weight of lysine which greatly improves the nutritional quality of the protein source. In one particular embodiment, the proteins of the composition are hydrolysed, fully hydrolysed or partially hydrolysed. The degree of hydrolysis (DH) of the protein can be between 2 and 20, or between 8 and 40, or between 20 and 60 or between 20 and 80 or more than 10, 20, 40, 60, 80 or 90. At least 70%, 80%, 85%, 90%, 95% or 97% of the proteins may be hydrolysed. In a particular embodiment, 100% of the proteins are hydrolysed.

A nutritional composition according to the present invention, and especially an infant formula of the invention, may contain a carbohydrate source. In this case, any carbohydrate source conventionally found in infant formulae such as lactose, sucrose, saccharose, maltodextrin, starch and mixtures thereof may be used although one of the preferred sources of carbohydrates for infant formula is lactose. The carbohydrate may be in an amount of from about 8 to about 15 g/100kcal or from about 9 to about 14 g/100kcal. In some embodiments, the carbohydrate is present in an amount of from about 10 to about 13 g/100kcal, or about 11.1 g/100kcal.

A nutritional composition according to the present invention, and especially an infant formula of the invention, may contain lipids and essential fatty acids. Non-limiting examples of lipids include palm olein, high oleic sunflower oil, high oleic safflower oil, canola oil, fish oil, coconut oil, bovine milk fat, and combinations thereof. Non-limiting examples of essential fatty acids include linoleic acid (LA), α-linolenic acid (ALA). Compositions of the invention may further contain gangliosides monosialoganglioside-3 (GM3) and disialogangliosides 3 (GD3), and combinations thereof. The lipid may be in an amount of from about 3.0 to about 8.0 g/100kcal, from about 4.0 to about 6.0 g/100kcal, or from about 4.5 to about 5.5 g/100kcal. In some embodiments, the lipid is present in an amount of from about 5.0 to about 5.5 g/100kcal, or about 5.3 g/100kcal.

A nutritional composition of the invention, and especially an infant formula of the invention, may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B3, vitamin B6, vitamin B12, vitamin E, vitamin K1, vitamin K2, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population. If necessary, a nutritional composition of the invention may contain emulsifiers and stabilisers such as soy, lecithin, citric acid esters of mono- and diglycerides, and the like.

Suitably, a nutritional composition of the invention, and especially an infant formula of the invention, may have an energy density of from about 60 to about 72 kcal per 100 mL or about 67 kcal per 100 mL

### Preparation of compositions

The compositions according to the present invention may be prepared by any known or otherwise suitable manner.

For example, a nutritional composition, e.g. an infant formula, may be proposed by blending together a source of protein with a carbohydrate source and a lipid source in appropriate proportions. If used, emulsifiers may be included at this stage. Vitamins and minerals may be added at this stage, but may also be added later to avoid thermal degradation. Water, preferably water which has been subjected to reverse osmosis or deionized water, may then be added and mixed in to form a liquid mixture. The temperature of mixing is preferably room temperature, but may also be higher. The liquid mixture may then be thermally treated to reduce bacterial loads. The mixture may then be homogenized.

If it is desired to produce a powdered composition, the homogenized mixture may be dried in a suitable drying apparatus, such as a spray drier or freeze drier and converted into powder.

Processes used in the manufacture of formulae for infants and young children are based on the concept that the products must be nutritionally adequate and microbiologically safe to consume. Thus, steps that eliminate or restrict microbiological growth are central to production processes. The processing technology generally involves the preservation of an oil-in-water (o/w) emulsion by dehydration in the case of powder products or, sterilization in the case of ready-to-feed or concentrated liquid products. Powdered infant formula may be produced using various processes, such as dry blending dehydrated ingredients to constitute a uniform formula or hydrating and wet-mixing a mixture of macro-ingredients, such as fat, protein and carbohydrate ingredients and then evaporating and spray drying the resultant mixture. A combination of the two processes described above may be used where a base powder is first produced by wet-mixing and spray drying all or some of the macro-ingredients and then dry blending the remaining ingredients, including carbohydrate, minerals and vitamins and other micronutrients, to create a final formula. Liquid formulae are available in a ready-to-feed format or as a concentrated liquid, which requires dilution, normally 1:1, with water. The manufacturing processes used for these products are similar to those used in the manufacture of recombined milk.

If it is desired to produce a liquid infant formula, the homogenized mixture may be filled into suitable containers, preferably aseptically. However, the liquid composition may also be retorted in the container, suitable apparatus for carrying out the filling and retorting of this nature is commercially available.

### Dosage of HMOs

The mixture of HMOs may be administered in any dosage that is effective to induce an infant's gut microbiome trajectory to converge with that of a reference gut microbiome trajectory and remain or stay convergent with the reference gut microbiome trajectory. The effective dosage may vary depending on e.g. the weight and/or age of the infant.

If the mixture of HMOs is administered in the form of an infant formula, the infant formula may be administered as normal (e.g. based on the weight of the infant or child) and suitable amounts of an individual HMO e.g. of 2'FL, diFL, LNT, 3'-SL, 6'-SL may be based on the amounts found in human breast milk produced for an infant or child of the same age, in particular by a nutritionally replete mother. The amounts in the infant formula may vary depending on for example bioavailability of said HMOs from infant formula in comparison to human breastmilk. The exemplary concentration of HMOs described herein may refer to the concentration after the composition has been reconstituted e.g. with water.

The amounts of 2'FL, diFL, LNT, 3'-SL, 6'-SL in human breast milk may fall within the following ranges: 2'FL in an amount of about 0.5 to about 3 g/L (e.g. about 1.8 g/L); diFL in an amount of about 0.1 to about 0.5 g/L (e.g. about 0.26 g/L); LNT in an amount of about 0.05 to about 0.3 g/L (e.g. about 0.77 g/L); 3'SL in an amount of about 0.1 to about 0.4 g/L (e.g. about 0.22 g/L); and 6'SL in an amount of about 0.05 to about 0.75 g/L (e.g. about 0.47 g/L).

Suitably, the mixture of HMOs (e.g. 2'FL, diFL, LNT, 3'-SL, and/or 6'-SL) is administered in a total amount of about 0.1 g/day to about 10 g/day. Suitably, the mixture of HMOs is administered in a total amount of about 0.5 g/day or more, about 1.0 g/day or more, or about 1.5 g/day or more. Suitably, the mixture of HMOs is administered in a total amount of about 5.0 g/day or less, 4.5 g/day or less, 4.0 g/day or less, 3.5 g/day or less, 3.0 g/day or less, or 2.5 g/day or less. Suitably, the mixture of HMOs is administered in a total amount of from about 0.5 g/day to about 5.0 g/day, from about 1.0 g/day to about 3.0 g/day, or from about 1.4 g/day to about 2.5 g/day. In some embodiments, the mixture of HMOs is administered in a total amount of from about 1.2 g/day to about 1.8 g/day (e.g. about 1.46 g/day) or from about 2.0 g/day to about 3.0 g/day (e.g. about 2.44 g/day). In some embodiments, the mixture of HMOs is administered in a total amount of from about 1.2 g/day to about 1.8 g/day. In some embodiments, the mixture of HMOs is administered in a total amount of about 1.46 g/day.

Suitably, when the mixture of HMOs is administered in the form of a starter infant formula the mixture of HMOs is administered in a total amount of from about 0.5 g/day to about 5.0 g/day, from about 1.0 g/day to about 3.0 g/day, or from about 1.5 g/day to about 2.5 g/day (e.g. about 1.5 g/day or about 2.5 g/day). Suitably, when the mixture of HMOs is administered in the form of a follow-up formula the mixture of HMOs is administered in a total amount of from about 0.1 g/day to about 2.0 g/day, from about 0.2 g/day to about 1.0 g/day, from about 0.3 g/day to about 0.7 g/day, or about 0.5 g/day. Suitably, when the mixture of HMOs is administered in the form of a growing-up milk the mixture of HMOs is administered in a total amount of from about 0.05 g/day to about 0.5 g/day, from about 0.1 g/day to about 0.3 g/day, or about 0.2 g/day.

Suitably, the mixture of HMOs is administered in the following proportions: (i) 2'FL in an amount of from about 55 wt% to about 60 wt% (e.g. about 58%); (ii) diFL in an amount of from about 5 wt% to about 7 wt% (e.g. about 6 wt%); (iii) LNT in an amount of from about 18 wt% to about 20 wt% (e.g. about 19 wt%); (iv) 3'-SL in an amount of from about 6 wt% to about 8 wt% (e.g. about 7 wt%); and (v) 6'-SL in an amount of from about 9 wt% to about 11 wt% (e.g. about 10 wt%), based on the total weight of HMOs.

### Concentration of HMOs

A nutritional composition (e.g. infant formula) comprising the mixture of HMOs may comprise the mixture of HMOs in any suitable concentrations to provide an effective dosage.

As a guide, for e.g. an infant formula, the mixture of HMOs (e.g. 2'FL, diFL, LNT, 3'-SL, and/or 6'-SL) may be present in a total amount of about 0.1 g/L to about 10 g/L. Suitably, the composition comprises the mixture of HMOs in a total amount of about 0.5 g/L or more, about 1.0 g/L or more, or about 1.5 g/L or more. Suitably, the composition comprises the mixture of HMOs in a total amount of about 5.0 g/L or less, 4.5 g/L or less, 4.0 g/L or less, 3.5 g/L or less, 3.0 g/L or less, or 2.5 g/L or less. Suitably, the composition comprises the mixture of HMOs in a total amount of from about 0.5 g/L to about 5.0 g/L, from about 1.0 g/L to about 3.0 g/L, from about 1.2 g/L to 3.0 g/L, or from about 1.5 g/L to about 2.5 g/L. In some embodiments, the composition comprises the mixture of HMOs in a total amount of from about 1.2 g/L to about 1.8 g/L (e.g. about 1.5 g/L) or from about 2.0 g/L to about 3.0 g/L (e.g. about 2.5 g/L). In some embodiments, the composition comprises the mixture of HMOs in a total amount of from about 1.2 g/L to about 1.8 g/L. In some embodiments, the composition comprises the mixture of HMOs in a total amount of about 1.5 g/L.

Suitably, a starter infant formula comprises a total amount of HMOs of from about 0.5 g/L to about 5.0 g/L, from about 1.0 g/L to about 3.0 g/L, from about 1.2 g/L to 3.0 g/L, or from about 1.5 g/L to about 2.5 g/L. Suitably, a follow-up formula comprises a total amount of HMOs of from about 0.1 g/L to about 2.0 g/L, from about 0.2 g/L to about 1.0 g/L, from about 0.3 g/L to about 0.8 g/L, from about 0.35 g/L to about 0.65 g/L, or about 0.5 g/L. Suitably, a growing-up milk comprises a total amount of HMOs of from about 0.1 g/L to about 1.0 g/L, from about 0.2 g/L to about 0.8 g/L, from about 0.28 g/L to about 0.52 g/L, from about 0.3 g/L to about 0.5 g/L, or about 0.4 g/L.

Suitably, the mixture of HMOs (e.g. in the form of a nutritional composition, such as an infant formula) comprises or consists of: (i) 2'FL in an amount of from about 50 wt% to about 65 wt%; (ii) diFL in an amount of from about 2 wt% to about 10 wt%; (iii) LNT in an amount of from about 15 wt% to about 25 wt%; (iv) 3'-SL in an amount of from about 4 wt% to about 10 wt%; and (v) 6'-SL in an amount of from about 5 wt% to about 15 wt%, based on the total weight of HMOs.

In some embodiments, the mixture of HMOs (e.g. in the form of a nutritional composition, such as an infant formula) comprises or consists of: (i) 2'FL in an amount of from about 55 wt% to about 60 wt% (e.g. about 58%); (ii) diFL in an amount of from about 5 wt% to about 7 wt% (e.g. about 6 wt%); (iii) LNT in an amount of from about 18 wt% to about 20 wt% (e.g. about 19 wt%); (iv) 3'-SL in an amount of from about 6 wt% to about 8 wt% (e.g. about 7 wt%); and (v) 6'-SL in an amount of from about 9 wt% to about 11 wt% (e.g. about 10 wt%), based on the total weight of HMOs.

As a guide, for e.g. an infant formula, the one or more fucosylated oligosaccharide (e.g. 2'FL and/or diFL) may be present in a total amount of from about 0.1 g/L to about 4 g/L. Suitably, the one or more fucosylated oligosaccharide is present in amount of about 0.1 g/L to about 3.5 g/L, about 0.15 g/L to about 3 g/L, from about 0.2 g/L to about 2.5 g/L, from about 0.3 g/L to about 2 g/L, from about 0.4 g/L to about 2 g/L, or from about 0.5 g/L to about 2 g/L. In some embodiments, 2'FL is present in an amount of from about 0.5 g/L to about 3.0 g/L (e.g. about 0.87 g/L or about 1.45 g/L). In some embodiments, 2'FL is present in an amount of about 0.87 g/L. In some embodiments, diFL is present in an amount of from about 0.05 g/L to about 0.3 g/L (e.g. about 0.10 g/L or about 0.14 g/L). In some embodiments, diFL is present in an amount of about 0.10 g/L.

As a guide, for e.g. an infant formula, the one or more N-acetylated oligosaccharide (e.g. LNT) may be present in a total amount of about 0.05 g/L to about 1.0 g/L. Suitably, the one or more N-acetylated oligosaccharide is present in amount of about 0.1 g/L to about 0.5 g/L, or about 0.2 g/L to about 0.5 g/L. In some embodiments, LNT is present in an amount of from about 0.1 g/L to about 1.0 g/L (e.g. about 0.29 g/L or about 0.48 g/L). In some embodiments, LNT is present in an amount of about 0.29 g/L.

As a guide, for e.g. an infant formula, the one or more sialylated oligosaccharide (e.g. 3'SL and/or 6'SL) may be present in a total amount of from about 0.05 g/L to about 1 g/L. Suitably, the one or more sialylated oligosaccharide is present in amount of about 0.05 g/L to about 0.5 g/L, or about 0.1 g/L to about 0.5 g/L. In some embodiments, 3'SL is present in an amount of from about 0.05 g/L to about 0.3 g/L (e.g. about 0.11 g/L or about 0.18 g/L). In some embodiments, 3'SL is present in an amount of about 0.11 g/L. In some embodiments, 6'SL is present in an amount of from about 0.05 g/L to about 0.5 g/L (e.g. about 0.14 g/L or about 0.24 g/L). In some embodiments, 6'SL is present in an amount of about 0.14 g/L.

Suitably, the mixture of HMOs (e.g. in the form of a nutritional composition, such as an infant formula) may comprise or consist of: (i) 2'FL in an amount of from about 0.5 g/L to about 3.0 g/L (e.g. about 0.87 g/L or about 1.45 g/L); (ii) diFL in an amount of from about 0.05 g/L to about 0.3 g/L (e.g. about 0.10 g/L or about 0.14 g/L); (iii) LNT in an amount of from about 0.1 g/L to about 1.0 g/L (e.g. about 0.29 g/L or about 0.48 g/L); (iv) 3'-SL in an amount of from about 0.05 g/L to about 0.3 g/L (e.g. about 0.11 g/L or about 0.18 g/L); and (v) 6'-SL in an amount of from about 0.05 g/L to about 0.5 g/L (e.g. about 0.14 g/L or about 0.24 g/L).

In some embodiments, the mixture of HMOs (e.g. in the form of a nutritional composition, such as a starter infant formula) comprises or consists of: (i) 2'FL in an amount of from about 0.70 g/L to about 1.05 g/L, preferably about 0.87 g/L; (ii) diFL in an amount of from about 0.05 g/L to about 0.11 g/L, preferably about 0.10 g/L; (iii) LNT in an amount of from about 0.23 g/L to about 0.36 g/L, preferably about 0.29 g/L; (iv) 3'-SL in an amount of from about 0.09 g/L to about 0.13 g/L, preferably about 0.11 g/L; and (v) 6'-SL in an amount of from about 0.12 g/L to about 0.17 g/L, preferably about 0.14 g/L.

In other embodiments, the mixture of HMOs (e.g. in the form of a nutritional composition, such as a starter infant formula) comprises or consists of: (i) 2'FL in an amount of from about 1.16 g/L to about 1.74 g/L, preferably about 1.45 g/L; (ii) diFL in an amount of from about 0.12 g/L to about 0.18 g/L, preferably about 0.14 g/L; (iii) LNT in an amount of from about 0.39 g/L to about 0.58 g/L, preferably about 0.48 g/L; (iv) 3'-SL in an amount of from about 0.14 g/L to about 0.21 g/L, preferably about 0.18 g/L; and (v) 6'-SL in an amount of from about 0.19 g/L to about 0.28 g/L, preferably about 0.24 g/L.

In other embodiments, the mixture of HMOs (e.g. in the form of a nutritional composition, such as a follow-up formula) comprises or consists of: (i) 2'FL in an amount of from about 0.19 g/L to about 0.34 g/L, preferably about 0.26 g/L; (ii) diFL in an amount of from about 0.03 g/L to about 0.05 g/L, preferably about 0.04 g/L; (iii) LNT in an amount of from about 0.06 g/L to about 0.11 g/L, preferably about 0.09 g/L; (iv) 3'-SL in an amount of from about 0.04 g/L to about 0.09 g/L, preferably about 0.06 g/L; and (v) 6'-SL in an amount of from about 0.03 g/L to about 0.06 g/L, preferably about 0.05 g/L.

In other embodiments, the mixture of HMOs (e.g. in the form of a nutritional composition, such as a growing-up milk) comprises or consists of: (i) 2'FL in an amount of from about 0.15 g/L to about 0.28 g/L, preferably about 0.21 g/L; (ii) diFL in an amount of from about 0.01 g/L to about 0.04 g/L, preferably about 0.03 g/L; (iii) LNT in an amount of from about 0.05 g/L to about 0.09 g/L, preferably about 0.07 g/L; (iv) 3'-SL in an amount of from about 0.03 g/L to about 0.08 g/L, preferably about 0.06 g/L; and (v) 6'-SL in an amount of from about 0.03 g/L to about 0.05 g/L, preferably about 0.04 g/L.

### Period of administration

The mixture of HMOs may be administered for any suitable period. For example, if administered at least in the form of a starter infant formula, the HMOs may be administered until at least about 6 months after birth. For example, if administered at least in the form of a starter infant formula and a follow-up formula, the HMOs may be administered until at least about 12 months after birth. For example, if administered at least in the form of a starter infant formula, a follow-up formula, and a growing-up milk, the HMOs may be administered until at least about 15 months after birth.

The mixture of HMOs may be administered at least from about 0 months to about 6 months after birth. Suitably, the mixture of HMOs is administered starting about 7 to about 21 days after birth. Suitably, the mixture of HMOs is administered until about 6 months to about 18 months after birth, until about 6 months to about 15 months after birth, or until about 6 months to about 12 months after birth. In some embodiments, the mixture of HMOs is administered starting about 7 to about 21 days after birth until about 15 months after birth, starting about 7 to about 21 days after birth until about 12 months after birth, starting about 7 to about 21 days after birth until about 9 months after birth, or starting about 7 to about 21 days after birth until about 6 months after birth.

### Infant of interest

The mixture of HMOs may be administered to any infant who has or is at risk of having a gut microbiome trajectory that differs from a reference gut microbiome (e.g. a reference gut microbiome from full-term vaginally-delivered and exclusively human milk-fed infants).

The infant may be formula-fed. As used herein, a "formula-fed" infant may refer to an infant who receives all or some of their nutrition during early infancy from infant formula (e.g. starter infant formula). In some embodiments, the infant receives all or substantially all of their nutrition during early infancy from infant formula. An infant's feeding mode has well-recognized effects on the gut microbiome composition and function.

The infant may be full-term or preterm. As used herein, a "preterm infant" may refer to an infant born before about 37 weeks of gestation. As used herein, a "full-term infant" may refer to an infant born at about 37 weeks of gestation or later. In some embodiments, the infant is full-term.

The infant may be vaginally-delivered or delivered by caesarean section. The inventors have shown that the benefits obtained with the present invention may be more pronounced in formula-fed infants who were delivered by caesarean section. In some embodiments, the infant is delivered by caesarean section.

Suitably, the infant is from about 0 years to about 2 years of age, from about 0 years to about 1 year of age, or from about 0 years to about 0.5 years of age. Suitably, the infant is from about 0 months to about 24 months of age, from about 0 months to about 18 months of age, from about 0 months to about 15 months of age, from about 0 months to about 12 months of age, from about 0 months to about 9 months of age, or from about 0 months to about 6 months of age.

### Gut microbiome trajectories

The present invention provides methods of inducing an infant's gut microbiome trajectory to converge with that of a reference gut microbiome trajectory and remain or stay convergent with the reference gut microbiome trajectory. The methods may comprise administering any mixture of HMOs described herein to the infant.

The "gut microbiota" is the composition of microorganisms (including bacteria, archaea and fungi) that live in the digestive tract. The term "gut microbiome" may encompass both the "gut microbiota" and their "theatre of activity", which may include their structural elements (nucleic acids, proteins, lipids, polysaccharides), metabolites (signalling molecules, toxins, organic, and inorganic molecules), and molecules produced by coexisting hosts and structured by the surrounding environmental conditions (see e.g. Berg, G., et al., 2020. Microbiome, 8(1), pp.1-22). In the present invention, the term "gut microbiome" may be used interchangeably with the term "gut microbiota".

A subject's "gut microbiome age" can refer to the predicted age of a subject based on their gut microbiome data. For example, the actual/chronological age of subject may be predicted from gut microbiome data obtained from a fecal sample using machine learning based Artificial Intelligence approaches. The term "gut microbiome age" encompasses both a subject's "gut microbiome compositional age" and "gut microbiome functional age". A "gut microbiome compositional age" may refer to a gut microbiome age which is determined using gut microbial composition data such as at genus-level or species-level composition data. A "gut microbiome functional age" may refer to a gut microbiome age which is determined using gut functional data such as pathway modules/submodules or metabolites data (e.g. CAZyme abundances). A subject's "gut microbiome maturation index" or "gut microbiome maturation age" may be obtained as above, from gut compositional or functional data, or obtained from gut compositional and functional data (and other similar data).

A subject's "gut microbiome diversity" can refer to the diversity of the subject's gut microbiome. A subject's gut microbiome diversity may refer to the number of different taxa present in the gut microbiome of the subject (e.g. "richness"). It may also refer to the "evenness" of the gut microbiome, i.e. takes into account the abundance or relative abundance of each taxon. Suitably, a subject's gut microbiome diversity can refer to the alpha diversity of the subject's gut microbiome. Alpha diversity may be the diversity of a single sample (such as a fecal sample), and can take into account the number of different taxa and their relative abundances.

A "gut microbiome trajectory" may refer to a fitted curve which describes the relation of "gut microbiome age", "gut microbiome maturation index", "gut microbiome maturation age" or "gut microbiome diversity" with age and may encompass gut microbiome age trajectories and gut microbiome diversity trajectories. The curve may be fitted by methods such as LOESS or smooth splines using another cohort or subset of data (for external validation purposes). Any suitable method may be used to provide a gut microbiome trajectory (see e.g. Dogra, S.K., Banjac, J. and Sprenger, N., 2022. bioRxiv 2022.02.14.479826).

A "reference gut microbiome trajectory" (e.g. an age predictor) may be obtained by training a regression model on or determining a relationship from gut microbiome data from a reference population. The reference population may be any suitable reference population. In some embodiments, the reference population is exclusively human milk-fed infants. In some embodiments, the reference population is vaginally-delivered and exclusively human milk-fed infants. In some embodiments, the reference population is full-term vaginally-delivered and exclusively human milk-fed infants. In some embodiments, the reference population is predominantly human milk-fed infants. In some embodiments, the reference population is vaginally-delivered and predominantly human milk-fed infants. In some embodiments, the reference population is full-term vaginally-delivered and predominantly human milk-fed infants.

### Gut microbiome age trajectories

In some embodiments, the present invention provides methods of inducing an infant's gut microbiome age trajectory to converge with that of a reference gut microbiome age trajectory.

A "gut microbiome age trajectory" may refer to a fitted curve which describes the relation of "gut microbiome age", "gut microbiome maturation index", or "gut microbiome maturation age" with actual age. A reference "gut microbiome age trajectory" may also be referred to as an "age predictor".

Any suitable method may be used to provide a reference gut microbiome age trajectory. For example, a method for providing a reference gut microbiome age trajectory may comprise:
(a) providing gut microbiome data from a reference population; and
(b) training a regression model on the gut microbiome data, wherein the age of the reference population at data collection is regressed on one or more features provided from the gut microbiome data.

Any suitable feature obtained from the gut microbiome data may be used to train the regression model. In some embodiments, the one or more features comprises or consists of one or more microbial abundances, one or more microbial ratios, or one or more carbohydrate-active enzyme (CAZyme) abundances. The features may be transformed in any way suitable for training a regression model (e.g. log-transformed).

In some embodiments, the one or more features comprises or consists of one or more microbial abundances. As used herein, a "microbial abundance" may refer to the relative abundance of a microbial taxon or the absolute abundance of a microbial taxon.

In some embodiments, the one or more features comprises or consists of one or more microbial ratios. As used herein, a "microbial ratio" refers to a ratio of the abundance of one microbial taxon to the abundance of another microbial taxon.

The microbial taxa may be classified according to any suitable classification, see e.g. Pitt, T.L. and Barer, M.R., 2012. Medical Microbiology, p.24. The microbial taxa may be classified by the same classification system(s) or by one or more different classification systems. The microbial taxa may be taxonomically-classified and/or functionally-classified.

In some embodiments, the microbial taxa are taxonomically-classified. Microbial taxonomy refers to the rank-based classification of microbes. In the scientific classification established by Carl Linnaeus, each species has to be assigned to a genus, which in turn is a lower level of a hierarchy of ranks (family, suborder, order, subclass, class, division/phyla, kingdom, and domain). Prokaryotic taxa which have been correctly described are reviewed in e.g. Bergey's manual of Systematic Bacteriology. Suitably, the microbial taxa are taxonomically-classified by phylum, class, order, family, genus and/or species. Suitably, the microbial taxa are taxonomically-classified by phylum, genus and/or species. Suitably, the microbial taxa are taxonomically-classified by genus and/or species. In some embodiments, the microbial taxa are taxonomically-classified by genus. In some embodiments, the microbial taxa are taxonomically-classified by species.

In some embodiments, the microbial taxa are functionally-classified. For example, the microbial taxa may be classified by one or more phenotypic classification systems (e.g. gram stain, morphology, growth requirements, biochemical reactions, serologic systems, environmental reservoirs etc). In some embodiments, the microbial taxa are classified according to biological or metabolic pathways, protein domains or families, functional modules, complex carbohydrate metabolism, antibiotic resistance, virulence factors, bacterial drug targets and endotoxins, mobile genetic elements, and/or any other functional properties, such as those described in Kultima, J.R., et al., 2016. Bioinformatics, 32(16), pp.2520-2523 and Overbeek, R., et al., 2014. Nucleic acids research, 42(D1), pp.D206-D214.

Suitable microbial taxa may be determined by any suitable method. For example, the suitability of a microbial taxon can be based on modelling performance statistics, availability or ease of testing, or on the infant of interest. Suitably, the microbial taxa are bacterial taxa. Any suitable bacterial taxa may be used, see e.g. Rinninella, E., et al., 2019. Microorganisms, 7(1), p.14. For example, (e.g. if the microbial taxa are taxonomically-classified by genus) the microbial taxa may comprise one or more bacterial taxa selected from *Escherichia, Roseburia, Faecalibacterium, Sutterella, SMB53, Collinsella, Ruminococcus, Akkermansia, Veillonella, Parabacteroides, Clostridium, Oscillospira, Megasphaera, Fusobacterium, Bacteroides, Citrobacter, Neisseria, Bifidobacterium, Lachnospira, Dialister, Ruminococcus, Blautia, Streptococcus, Eggerthella, Paraprevotella, Corynebacterium, Atopobium, Lactobacillus, Enterococcus, Staphylococcus, Sphingobacterium, Tannerella, Alistipes, Prevotella, Shigella, Desulfovibrio, Bilophila,* and *Helicobacter*. For example, (e.g. if the microbial taxa are taxonomically-classified by species) the microbial taxa may comprise one or more bacterial taxa selected from *Bifidobacterium longum, Bifidobacterium bifidum, Faecalibacterium prausnitzii, Clostridium spp., Roseburia intestinalis, Ruminococcus faecis, Dialister invisus, Lactobacillus reuteri, Enterococcus faecium, Staphylococcus leei, Bacteroides fragilis, Bacteroides vulgatus, Bacteroides uniformis, Parabacteroides distasonis, Alistipes finegoldii, Prevotella spp., Escherichia coli, Shigella flexneri, Desulfovibrio intestinalis, Helicobacter pylori, Fusobacterium nucleatum,* and *Akkermansia muciniphilia*.

In some embodiments, the one or more features comprises or consists of one or more CAZyme abundances. As used herein, a "CAZyme abundance" may refer to the abundance of a CAZyme gene in gut microbiome data. The abundance may be a relative abundance and/or absolute abundance. Suitably, abundance is a relative abundance, for example, the abundances may be calculated relative to total bacterial genes (see e.g. Kaur, K., et al., 2020. PloS one, 15(4), p.e0231197) or relative to total CAZyme abundance. Suitably, the abundances are calculated relative to total bacterial genes.

Carbohydrate-active enzymes (CAZymes) may refer to enzymes involved in the synthesis, metabolism, and transport of carbohydrates. CAZymes may include glycoside hydrolases (GHs), glycosyltransferases (GTs), polysaccharide lyases (PLs), carbohydrate esterases (CEs) and carbohydrate binding modules (CBMs). Suitably, the CAZymes are microbial CAZymes. The CAZymes may be classified according to any suitable classification system, see e.g. Lombard, V., et al., 2014. Nucleic acids research, 42(D1), pp.D490-D495. The CAZymes may be classified by the same classification system(s) or by one or more different classification systems. Suitably, the CAZymes are classified by clans, families, and/or subfamilies. Suitably, the CAZymes are classified by families.

Suitably, the CAZymes comprise, consist essentially of, or consist of one or more (e.g. 5 or more, 10 or more, 20 or more, 50 or more, or 100 or more) of: GH1, GH2, GH3, GH4, GH5, GH6, GH7, GH8, GH9, GH10, GH11, GH12, GH13, GH14, GH15, GH16, GH17, GH18, GH19, GH20, GH21, GH22, GH23, GH24, GH25, GH26, GH27, GH28, GH29, GH30, GH31, GH32, GH33, GH34, GH35, GH36, GH37, GH38, GH39, GH40, GH41, GH42, GH43, GH44, GH45, GH46, GH47, GH48, GH49, GH50, GH51, GH52, GH53, GH54, GH55, GH56, GH57, GH58, GH59, GH60, GH61, GH62, GH63, GH64, GH65, GH66, GH67, GH68, GH69, GH70, GH71, GH72, GH73, GH74, GH75, GH76, GH77, GH78, GH79, GH80, GH81, GH82, GH83, GH84, GH85, GH86, GH87, GH88, GH89, GH90, GH91, GH92, GH93, GH94, GH95, GH96, GH97, GH98, GH99, GH100, GH101, GH102, GH103, GH104, GH105, GH106, GH107, GH108, GH109, GH110, GH111, GH112, GH113, GH114, GH115, GH116, GH117, GH118, GH119, GH120, GH121, GH122, GH123, GH124, GH125, GH126, GH127, GH128, GH129, GH130, GH131, GH132, GH133, GH134, GH135, GH136, GH137, GH138, GH139, GH140, GH141, GH142, GH143, GH144, GH145, GH146, GH147, GH148, GH149, GH150, GH151, GH152, GH153, GH154, GH155, GH156, GH157, GH158, GH159, GH160, GH161, GH162, GH163, GH164, GH165, GH166, GH167, GH168, GH169, GH170, GH171, GH172, GT1, GT2, GT3, GT4, GT5, GT6, GT7, GT8, GT9, GT10, GT11, GT12, GT13, GT14, GT15, GT16, GT17, GT18, GT19, GT20, GT21, GT22, GT23, GT24, GT25, GT26, GT27, GT28, GT29, GT30, GT31, GT32, GT33, GT34, GT35, GT36, GT37, GT38, GT39, GT40, GT41, GT42, GT43, GT44, GT45, GT46, GT47, GT48, GT49, GT50, GT51, GT52, GT53, GT54, GT55, GT56, GT57, GT58, GT59, GT60, GT61, GT62, GT63, GT64, GT65, GT66, GT67, GT68, GT69, GT70, GT71, GT72, GT73, GT74, GT75, GT76, GT77, GT78, GT79, GT80, GT81, GT82, GT83, GT84, GT85, GT86, GT87, GT88, GT89, GT90, GT91, GT92, GT93, GT94, GT95, GT96, GT97, GT98, GT99, GT100, GT101, GT102, GT103, GT104, GT105, GT106, GT107, GT108, GT109, GT110, GT111, GT112, GT113, GT114, PL1, PL2, PL3, PL4, PL5, PL6, PL7, PL8, PL9, PL10, PL11, PL12, PL13, PL14, PL15, PL16, PL17, PL18, PL19, PL20, PL21, PL22, PL23, PL24, PL25, PL26, PL27, PL28, PL29, PL30, PL31, PL32, PL33, PL34, PL35, PL36, PL37, PL38, PL39, PL40, PL41, PL42, CE1, CE2, CE3, CE4, CE5, CE6, CE7, CE8, CE9, CE10, CE11, CE12, CE13, CE14, CE15, CE16, CE17, CE18, CE19, CBM1, CBM2, CBM3, CBM4, CBM5, CBM6, CBM7, CBM8, CBM9, CBM10, CBM11, CBM12, CBM13, CBM14, CBM15, CBM16, CBM17, CBM18, CBM19, CBM20, CBM21, CBM22, CBM23, CBM24, CBM25, CBM26, CBM27, CBM28, CBM29, CBM30, CBM31, CBM32, CBM33, CBM34, CBM35, CBM36, CBM37, CBM38, CBM39, CBM40, CBM41, CBM42, CBM43, CBM44, CBM45, CBM46, CBM47, CBM48, CBM49, CBM50, CBM51, CBM52, CBM53, CBM54, CBM55, CBM56, CBM57, CBM58, CBM59, CBM60, CBM61, CBM62, CBM63, CBM64, CBM65, CBM66, CBM67, CBM68, CBM69, CBM70, CBM71, CBM72, CBM73, CBM74, CBM75, CBM76, CBM77, CBM78, CBM79, CBM80, CBM81, CBM82, CBM83, CBM84, CBM85, CBM86, CBM87, and CBM88.

Any suitable number of features may be used to train the regression model. Suitably, 5 or more, 6 or more, 7 or more, 8 or more, 9 or more, or 10 or more features may be used to train the regression model. For example, the regression model may be trained using 10 microbial abundances (e.g. if the microbial taxa are taxonomically-classified by genus), 20 or 25 microbial abundances (e.g. if the microbial taxa are taxonomically-classified by species), or 30 CAZyme abundances.

### Gut microbiome diversity trajectories

In some embodiments, the present invention provides methods of inducing an infant's gut microbiome diversity trajectory to converge with that of a reference gut microbiome diversity trajectory. A "gut microbiome diversity trajectory" may refer to a fitted curve which is obtained to describe the relation of gut diversity with age.

Any suitable method may be used to provide a reference gut microbiome diversity trajectory. For example, a method for providing a reference gut microbiome age diversity trajectory may comprise:
(a) providing gut microbiome data from a reference population; and
(b) determining the relationship between the average gut microbiome diversity of the reference population and the age of the reference population at data collection.

Alpha diversity can be determined using a richness index, a phylogenetic diversity index, or a Shannon index. These indexes can be determined using methods routine in the art, such as, for example, using the R package Phyloseq (McMurdie and Holmes, 2013, PLoS One, 8, Article e61217). Alpha diversity indexes may be calculated based on 16 rRNA sequencing data and/or whole genome shotgun metagenomics sequencing. Beta diversity can be determined using a Whittaker index (e.g. Jaccard or Sorensen), a Min-Max Index (e.g. Simpson, β-2 or β-3), a Cody Index or an Abundance index (e.g. Bray-Curtis or BD_{TOTAL}). Beta diversity indexes may be calculated based on 16 rRNA sequencing data and/or whole genome shotgun metagenomics sequencing. These indexes can be determined using methods routine in the art, such as, for example, using the R package Phyloseq (see e.g. McMurdie and Holmes, 2013, PLoS One, 8, Article e61217).

The microbial taxa used to determine the gut microbiome diversity may be classified according to any suitable classification described herein. Suitably, the microbial taxa are taxonomically-classified by genus and/or species. In some embodiments, the microbial taxa are taxonomically-classified by genus. In some embodiments, the microbial taxa are taxonomically-classified by species.

### Gut microbiome data

A reference gut microbiome trajectory (e.g. a reference gut microbiome age trajectory or a reference gut microbiome diversity trajectory) may be determined using gut microbiome data from a reference population.

The gut microbiome data may be any data suitable for determining a reference gut microbiome trajectory, for example the microbiome data may include gut microbial abundances, gut metagenomic data, gut metabolite data etc. Suitably, the gut microbiome data is gut metagenomic data. A subject's "gut metagenomic data" or "gut metagenome data" may refer to all the genetic content of the subject's gut, including all the genomes and genes from the gut microbiota (see e.g. Berg, G., et al., 2020. Microbiome, 8(1), pp.1-22; Pasolli, E., et al., 2019. Cell, 176(3), pp.649-662; and Qin, J., et al., 2010. Nature, 464(7285), pp.59-65).

The gut microbiome data may be obtained or obtainable by any suitable sampling method. For example, gut microbiome data may be obtained or obtainable by any method described in Tang, Q., et al., 2020. Frontiers in cellular and infection microbiology, 10, p.151. The gut microbiome data may be obtained from or obtainable from fecal samples, endoscopy samples (e.g. biopsy samples, luminal brush samples, laser capture microdissection samples), aspirated intestinal fluid samples, surgery samples, or by in vivo models or intelligent capsule.

Suitably, the gut microbiome data may be obtained from or obtainable from fecal samples. Fecal samples are naturally collected, non-invasive and can be sampled repeatedly. Fecal materials instantly frozen at -80°C that can maintain microbial integrity without preservatives have been widely regarded as the gold standard for gut metagenomics, but other storage methods with or without preservatives can also be utilised to achieve metagenomic data similar to those of fresh samples.

The gut microbiome data may be obtained by or obtainable from the samples by any suitable method. For example, the gut microbiome data may be obtained by or obtainable from the samples by sequencing methods (e.g. next-generation sequencing (NGS) methods). NGS enables the profiling of the genomic DNA of all the microorganisms present in a sample. NGS methods can include shotgun sequencing approaches, e.g. as described in Poussin, C., et al., 2018. Drug discovery today, 23(9), pp.1644-1657.

The reference population may comprise any number of infants suitable for training a regression model or determining a relationship. The reference population may comprise at least 10 infants, at least 20 infants, at least 30 infants, at least 40 infants, at least 50 infants, at least 60 infants, at least 80 infants, or at least 100 infants. Suitably, the reference population of may comprise 500 infants or less, 100 infants or less, or 50 infants or less. Suitably, the reference population may comprise from 10 to 500 infants.

The gut microbiome data from a reference population of may comprise any number of samples suitable for training a regression model. Suitably, the gut microbiome data comprises at least 50 samples, at least 100 samples, at least 200 samples, at least 300 samples, at least 400 samples, at least 500 samples, or at least 1000 samples.

The gut microbiome data from a reference population may comprise any number of samples from any number of infants which is suitable for training a regression model. Suitably, the gut microbiome data from a reference population may comprise at least 50 samples from at least 10 infants.

An infant's gut microbiome trajectory may be obtained or obtainable by any suitable sampling method described herein. The infant's gut microbiome data may be obtained or obtainable by the same method as the gut microbiome data from the reference population or by a different method.

### Regression analysis

A reference gut microbiome trajectory may be determined using regression analysis to relate the age of a reference population at data collection to one or more features provided from their metagenomic data.

Regression analysis is a set of statistical processes for estimating the relationships between a dependent variable (e.g. the age of the reference population of at data collection) and one or more independent variables (e.g. one or more features from the gut microbiome data). The regression analysis may be used to provide a trained (or fitted) regression model (i.e. a reference gut microbiome trajectory).

The regression analysis may be performed using be any suitable regression model. Suitable regression models will be well known to the skilled person. Exemplary regression models include decision tree regression, linear regression, polynomial regression, quantile regression, ridge regression, lasso regression, elastic net regression, and support vector regression.

Suitably, the regression analysis is performed using machine learning methods. Exemplary machine learning methods include tree-based regression models (e.g. a random forest regression models), recursive partitioning, regularized and shrinkage methods, boosting and gradient descent, and Bayesian methods. Suitably, the regression model is a tree-based regression model (e.g. a random forest regression model). In some embodiments, the regression model is a random forest regression model. In some embodiments, the regression model is a xgboost regression model.

The regression analysis may be performed by training a regression model on the gut microbiome data. For example, regression analysis may be performed by training a regression model using the age of the reference population at data collection and one or more features provided from the gut microbiome data.

As used herein, "training" of "fitting" a regression model may mean determining a function which most closely fits the data according to a suitable statistical criteria. For example, the method of ordinary least squares may be used to compute the function that minimizes the sum of squared differences between the true data and that function.

### Convergence of gut microbiome trajectories

The present inventors have shown in the present invention that a mixture of human milk oligosaccharides (HMOs) may induce an infant's gut microbiome trajectory to converge with that of a reference gut microbiome trajectory and remain or stay convergent with the reference gut microbiome trajectory.

As used herein, "convergent" gut microbiome trajectories tend to come closer together and meet at a point. Suitably, gut microbiome trajectories may be deemed to converge when they are no longer significantly statistically different. For example, an infant's gut microbiome trajectory may be deemed to converge with a reference gut microbiome trajectory if the infant is not an outlier in the reference gut microbiome trajectory and/or if the infant is on the reference gut microbiome trajectory. In some embodiments, the mixture of HMOs induce the infant's gut microbiome trajectory to remain or stay convergent with that of the reference gut microbiome trajectory.

As used herein, that the mixture of HMOs "induces" an infant's gut microbiome trajectory to converge with that of a reference gut microbiome trajectory may mean that the infant's gut microbiome trajectory converges with the reference gut microbiome trajectory earlier than an infant who is not administered the mixture of HMOs (i.e. in the absence of the mixture of HMOs). The term "induces" may be used interchangeably with the term "promotes". The phrase "inducing an infant's gut microbiome trajectory to converge with that of a reference gut microbiome trajectory" may be used interchangeably with the phrase "accelerating the convergence of an infant's gut microbiome trajectory with that of a reference gut microbiome trajectory".

The infant's gut microbiome trajectory may converge with that of the reference gut microbiome trajectory at least about 1 month, at least about 2 months, or at least about 3 months earlier compared to an infant who is not administered the mixture of HMOs (i.e. in the absence of the mixture of HMOs). The infant's gut microbiome trajectory may converge with that of the reference gut microbiome trajectory at least about 2 months earlier compared to an infant who is not administered the mixture of HMOs (i.e. in the absence of the mixture of HMOs).

Suitably, the infant's gut microbiome trajectory converges with that of the reference gut microbiome trajectory at about 12 months of age or earlier, about 11 months of age or earlier, about 10 months of age or earlier, about 9 months of age or earlier, at about 8 months of age or earlier, or at about 7 months of age or earlier.

Suitably, the infant's gut microbiome trajectory converges with that of the reference gut microbiome trajectory at about 6 months of age or later, at about 7 months of age or later, at about 8 months of age or later, at about 9 months of age or later, at about 10 months of age or later, at about 11 months of age or later, or at about 12 months of age or later.

Suitably, the infant's gut microbiome trajectory converges with that of the reference gut microbiome trajectory at about 6 to about 12 months of age, at about 6 to about 11 months of age, at about 6 to about 10 months of age, or at about 6 to about 9 months of age. Suitably, the infant's gut microbiome trajectory converges with that of the reference gut microbiome trajectory at about 7 to about 12 months of age, at about 7 to about 11 months of age, at about 7 to about 10 months of age, or at about 7 to about 9 months of age. Suitably, the infant's gut microbiome trajectory converges with that of the reference gut microbiome trajectory at about 8 to about 12 months of age, at about 8 to about 11 months of age, at about 8 to about 10 months of age, or at about 8 to about 9 months of age. Suitably, the infant's gut microbiome trajectory converges with that of the reference gut microbiome trajectory at about 8 to about 10 months of age, or at about 9 months of age.

### Outliers in reference gut microbiome trajectory

In some embodiments, the mixture of human milk oligosaccharides (HMOs) induces the infant to be not an outlier in the reference gut microbiome trajectory.

Any suitable statistical method may be used to determine whether the infant is an outlier in the reference gut microbiome trajectory (see e.g. Hodge, V. and Austin, J., 2004. Artificial intelligence review, 22(2), pp.85-126). For example, the infant may be determined to be an outlier based on the standard errors, confidence intervals, prediction intervals, and/or standard deviations in the reference gut microbiome trajectory. Suitably, the infant may be determined to be an outlier if their gut microbiome data differs significantly from the reference gut microbiome trajectory (e.g. age predictor) line, based on the standard errors, confidence intervals, prediction intervals, and/or standard deviations of the reference gut microbiome trajectory.

Suitable cut-offs will be well known to the skilled person. For example, three standard deviations from the mean is a common cut-off in practice for identifying outliers in a Gaussian or Gaussian-like distribution.

In some embodiments, the infant is determined to be an outlier based on the standard error of the reference gut microbiome trajectory. The standard error (SE) represents the average distance that the observed values fall from the reference gut microbiome trajectory. Suitably, the infant is an outlier if their gut microbiome data is -2SE or less or 2SE or more, -2.5SE or less or 2.5SE or more, -3SE or less or 3SE or more, -3.5SE or less or 3.5SE or more, or -4SE or less or 4SE or more from the reference gut microbiome trajectory. Suitably, the infant is an outlier if their gut microbiome data is -3SE or less or 3SE or more from the reference gut microbiome trajectory.

In some embodiments, the infant is determined to be an outlier based on the confidence interval of the reference gut microbiome trajectory. The confidence interval may be determined by any suitable method, for example using resampling approaches (e.g. bootstrap resampling). Suitably, the infant is an outlier if their gut microbiome data falls outside the 90% confidence interval, the 95% confidence interval, the 98% confidence interval, or the 99% confidence interval in the reference gut microbiome trajectory. Suitably, the infant is an outlier if their gut microbiome data falls outside the 95% confidence interval in the reference gut microbiome trajectory.

In some embodiments, the infant is determined to be an outlier based on prediction interval of the reference gut microbiome trajectory. Suitably, the infant is an outlier if their gut microbiome data falls outside the 90% prediction interval, the 95% prediction interval, the 98% prediction interval, or the 99% prediction interval in the reference gut microbiome trajectory. Suitably, the infant is an outlier if their gut microbiome data falls outside the 95% prediction interval in the reference gut microbiome trajectory.

In some embodiments, the infant is determined to be an outlier based on standard deviation of the reference gut microbiome trajectory. For example, a Z-score can be used to determine whether the infant is an outlier. The Z-score is the number of standard deviations above and below the mean. Suitably, the infant is an outlier if they have a Z-score of -2 or less or 2 or more, a Z-score of -2.5 or less or 2.5 or more, a Z-score of -3 or less or 3 or more, a Z-score of -3.5 or less or 3.5 or more, or a Z-score of -4 or less or 4 or more in the reference gut microbiome trajectory. Suitably, the infant is an outlier if they have a Z-score of -3 or less or 3 or more in the reference gut microbiome trajectory.

In some embodiments, the infant is determined to be an outlier if their gut microbiome data is -3SE or less or 3SE or more from the reference gut microbiome trajectory, if their gut microbiome data falls outside the 95% confidence interval in the reference gut microbiome trajectory, if their gut microbiome data falls outside the 95% prediction interval in the reference gut microbiome trajectory, and/or if they have a Z-score of -3 or less or 3 or more in the reference gut microbiome trajectory.

In some embodiments, the infant is determined to be an outlier if their gut microbiome data trajectory has a Z-score of -3 or less or 3 or more in the reference gut microbiome trajectory.

### On or off reference gut microbiome trajectory

In some embodiments, the mixture of human milk oligosaccharides (HMOs) induces the infant to be on the reference gut microbiome trajectory.

Suitably, the infant is on the reference gut microbiome trajectory if the infant's gut microbiome data does not differ significantly from the reference gut microbiome trajectory and/or the infant is off the reference gut microbiome trajectory if the infant's gut microbiome data differs significantly from the reference gut microbiome trajectory.

Any suitable method may be used to determine whether the infant is on the reference gut microbiome trajectory. For example, the infant may be determined to be on the reference gut microbiome trajectory based on the standard errors, confidence intervals, prediction intervals, and/or standard deviations of the reference gut microbiome trajectory.

In some embodiments, the infant is determined to be off the reference gut microbiome trajectory based on the standard error (SE) of the reference gut microbiome trajectory. Suitably, the infant is off the reference gut microbiome trajectory if their gut microbiome data is -2SE or less or 2SE or more, -2.5SE or less or 2.5SE or more, -3SE or less or 3SE or more, 3.5SE or less or 3.5SE or more, or -4SE or less or 4SE or more from the reference gut microbiome trajectory. Suitably, the infant is off the reference gut microbiome trajectory if their gut microbiome data is -3SE or less or 3SE or more from the reference gut microbiome trajectory.

In some embodiments, the infant is determined to be off the reference gut microbiome trajectory based on the confidence interval of the reference gut microbiome trajectory. Suitably, the infant is off the reference gut microbiome trajectory if their gut microbiome data falls outside the 90% confidence interval, the 95% confidence interval, the 98% confidence interval, or the 99% confidence interval of the reference gut microbiome trajectory. Suitably, the infant is off the reference gut microbiome trajectory if their gut microbiome data falls outside the 95% confidence interval of the reference gut microbiome trajectory.

In some embodiments, the infant is determined to be off the reference gut microbiome trajectory based on prediction interval of the reference gut microbiome trajectory. Suitably, the infant is off the reference gut microbiome trajectory if their gut microbiome data falls outside the 90% prediction interval, the 95% prediction interval, the 98% prediction interval, or the 99% prediction interval of the reference gut microbiome trajectory. Suitably, the infant is off the reference gut microbiome trajectory if their gut microbiome data falls outside the 95% prediction interval of the reference gut microbiome trajectory

In some embodiments, the infant is determined to be off the reference gut microbiome trajectory based on standard deviation of the reference gut microbiome trajectory. For example, a Z-score can be used to determine whether the infant is off the reference gut microbiome trajectory. Suitably, the infant is an outlier if they have a Z-score of -2 or less or 2 or more, a Z-score of -2.5 or less or 2.5 or more, a Z-score of -3 or less or 3 or more, a Z-score of -3.5 or less or 3.5 or more, or a Z-score of -4 or less or 4 or more. Suitably, the infant is off the reference gut microbiome trajectory if they have a Z-score of -3 or less or 3 or more.

In some embodiments, the infant is determined to be off the reference gut microbiome trajectory if their gut microbiome data is -3SE or less or 3SE or more from the reference gut microbiome trajectory, if their gut microbiome data falls outside the 95% confidence interval of the reference gut microbiome trajectory, if their gut microbiome data falls outside the 95% prediction interval of the reference gut microbiome trajectory, and/or if they have a Z-score of -3 or less or 3 or more.

In some embodiments, the infant is determined to be off the reference gut microbiome trajectory if they have a Z-score of -3 or less or 3 or more.

### Gut maturation

The methods of the present invention may promote gut maturation by inducing the infant's gut microbiome trajectory to converge with that of a reference gut microbiome trajectory.

Gut maturation during normal development involves a series of structural and functional changes that culminate during the weaning period when complex food is introduced. As used herein, "promoting" gut maturation may mean that the infant's gut matures in a more age-appropriate manner. Gut maturation may refer to maturation of the gut microbiome, maturation of gut metabolism, maturation of gut barrier function, and/or maturation of gut immune function.

The mixture of HMOs may promote maturation of the gut microbiome. Early gut microbiome maturation can be characterized by specific temporal microorganism acquisition, colonization and selection with differential functional features over time. This orchestrated microbial sequence occurs from birth during the first years of age before the microbiome reaches an adult-like composition and function between 3 and 5 years of age. Increasingly, these different steps of microbiome development are recognized as crucial windows of opportunity for long term health, primarily linked to appropriate immune and metabolic development (see e.g. Dogra, S.K., et al., 2021. Microorganisms, 9(10), p.2110). For example, maturation of the gut microbiome during the first year of life can contribute to the protective farm effect on childhood asthma (see e.g. Depner, M., et al., 2020. Nature medicine, 26(11), pp.1766-1775).

The mixture of HMOs may promote maturation of gut metabolism. Microbiome compositional changes also reflect to some extent microbial functional competencies, as illustrated by marked changes in the abundance of microbial carbohydrate active enzymes (CAZymes) and other metabolic pathways (see e.g. Stewart, C.J., et al., 2018. Nature, 562(7728), pp.583-588).

The mixture of HMOs may promote maturation of gut barrier function. The intestinal barrier built of mucus and underlying epithelial cells is primarily considered a physical barrier contributing together with numerous immune defence components to modulate the microbiome and host relationship (see e.g. Dogra, S.K., et al., 2021. Microorganisms, 9(10), p.2110).

The mixture of HMOs may promote maturation of gut immune function. Gut immune components, such as secretory immunoglobulin (Ig) A and defensins, together with epithelial and mucous glycosylation patterns change while the gut develops and likely play an important role in setting the stage for the development of host-microbiome mutualism (see e.g. Dogra, S.K., et al., 2021. Microorganisms, 9(10), p.2110).

The mixture of HMOs may modulate the abundance of one or more microbes and/or microbial metabolic pathways which are associated with age-appropriate gut maturation and associated health benefits. For example, HMO-stimulated bifidobacterium species may contribute to prevent later respiratory tract infections (see e.g. Dogra, S.K., et al., 2021. Microorganisms, 9(9), p.1939) and members of the Lachnospiraceae family, as well as the genera Faecalibacterium and Dialister, are associated with a reduced risk of atopy (see e.g. Galazzo, G., et al., 2020. Gastroenterology, 158(6), pp.1584-1596).

In one aspect, the present invention provides a mixture of HMOs to promote gut maturation in an infant by inducing the infant's gut microbiome trajectory to converge with that of a reference gut microbiome trajectory.

In one aspect, the present invention provides a method of promoting gut maturation in an infant in need thereof, wherein the method comprises administering a therapeutically effective amount of a mixture of HMOs, thereby inducing the infant's gut microbiome trajectory to converge with that of a reference gut microbiome trajectory.

### Method for determining the gut maturation status of an infant

The present invention also provides methods for determining the gut maturation status of an infant. Suitably, the methods comprise: (a) providing a reference gut microbiome trajectory; and (b) providing gut microbiome data from the infant and determining whether the infant is an outlier or not in the reference gut microbiome trajectory or whether the infant is on or off the reference gut microbiome trajectory.

The infant may be any suitable infant, for example any infant described in the section above titled "Infant of interest". Suitably, the infant is formula-fed. In some embodiments, the infant is full-term. In some embodiments, the infant is delivered by caesarean section. The infant may be administered a mixture of HMOs, for example as described in the section above titled "Human milk oligosaccharide (HMO) mixture".

The reference gut microbiome trajectory may be any suitable reference gut microbiome trajectory, for example any reference gut microbiome trajectory described in the section above titled "Gut microbiome trajectories". In some embodiments, the reference gut microbiome trajectory is obtained from full-term vaginally-delivered and exclusively human milk-fed infants.

Any suitable method may be used to determine whether the infant is an outlier or not in the reference gut microbiome trajectory or whether the formula-fed infant is on or off the reference gut microbiome trajectory, for example as described in the sections above titled "Outliers in reference gut microbiome trajectory" and "On or off reference gut microbiome trajectory".

Suitably, the gut maturation status of the infant is normal if the infant is not an outlier in the reference gut microbiome trajectory, and/or the gut maturation status of the infant is not normal if the infant is an outlier in the reference gut microbiome trajectory. In this context, a "normal" gut maturation status may mean that the infant has gut metagenome that does not differ significantly from the gut metagenome of a reference population.

### Computer program and computer-readable medium

The methods for determining the gut maturation status of an infant described herein may be computer-implemented methods.

In one aspect, the present invention provides a data processing system comprising means for carrying out a method for determining the gut maturation status of an infant described herein.

In one aspect, the present invention provides a data processing apparatus comprising a processor configured to perform a method for determining the gut maturation status of an infant described herein.

In one aspect, the present invention provides a computer program comprising instructions which, when the program is executed by a computer, cause the computer to carry out a method for determining the gut maturation status of an infant described herein.

In one aspect, the present invention provides a computer-readable medium comprising instructions which, when executed by a computer, cause the computer to carry out a method for determining the gut maturation status of an infant described herein.

In one aspect, the present invention provides a computer-readable data carrier having stored thereon the computer program of the invention.

In one aspect, the present invention provides a data carrier signal carrying the computer program of the invention.

In one aspect, the present invention provides a computer-implemented method for determining the gut maturation status of an infant, wherein the method comprises: (a) providing a reference gut microbiome trajectory and the microbiome-age predictor model associated with it; (b) providing gut microbiome data from the infant; and (c) determining whether the infant is an outlier or not in the reference gut microbiome trajectory; wherein the gut maturation status of the infant is normal if the infant is not an outlier in the reference gut microbiome trajectory, and/or wherein the gut maturation status of the infant is not normal if the infant is an outlier in the reference gut microbiome trajectory.

In one aspect, the present invention provides a computer-implemented method for determining the gut maturation status of an infant, wherein the method comprises: (a) providing a reference gut microbiome trajectory and the microbiome-age predictor model associated with it; (b) providing gut microbiome data from the infant; and (c) determining whether the infant is on or off the reference gut microbiome trajectory; wherein the infant is on the reference gut microbiome trajectory if the infant's gut microbiome data does not differ significantly from the reference gut microbiome trajectory and/or wherein the infant is off the reference gut microbiome trajectory if the infant's gut microbiome data differs significantly from the reference gut microbiome trajectory.

In one aspect, the present invention provides a data processing system comprising means for determining the gut maturation status of an infant given a reference gut microbiome trajectory, the microbiome-age predictor model associated with it and the infant's gut microbiome data, as described herein.

In one aspect, the present invention provides a data processing apparatus comprising a processor configured to determine the gut maturation status of an infant given a reference gut microbiome trajectory, the microbiome-age predictor model associated with it and the infant's gut microbiome data, as described herein.

In one aspect, the present invention provides a computer program comprising instructions which, when the program is executed by a computer, cause the computer to determine the gut maturation status of an infant given a reference gut microbiome trajectory, the microbiome-age predictor model associated with it and the infant's gut microbiome data, as described herein.

In one aspect, the present invention provides a computer-readable medium comprising instructions which, when executed by a computer, cause the computer to determine the gut maturation status of an infant given a reference gut microbiome trajectory, the microbiome-age predictor model associated with it and the infant's gut microbiome data, as described herein.

The systems described herein may display a dashboard or other appropriate user interface to a user that is customized based on the infant of interest. For example, based on the infant's gut metagenomic samples, the infant's determined gut maturation status, and the infant's personalized advice and recommendations such as HMO supplementation to maintain or improve the infant's gut maturation status.

### Use of gut microbiome trajectories

In another aspect, the present invention provides use of one or more reference gut microbiome trajectories to determine the gut maturation status of an infant following administration of a mixture of HMOs.

The infant may be any suitable infant, for example any infant described in the section above entitled "Infant of interest". Suitably, the infant is formula-fed. In some embodiments, the infant is full-term. In some embodiments, the infant is delivered by caesarean section. Any suitable mixture of HMOs may be administered to the infant, for example as described in the section above entitled "Human milk oligosaccharide (HMO) mixture".

The one or more reference gut microbiome trajectories may comprise or consist of any suitable reference gut microbiome trajectories, for example any reference gut microbiome trajectory described in the section above entitled "Gut microbiome trajectories". In some embodiments, the one or more reference gut microbiome trajectory is obtained from full-term vaginally-delivered and exclusively human milk-fed infants. In some embodiments, the one or more reference gut microbiome trajectory is obtained from full-term vaginally-delivered and predominantly human milk-fed infants.

The use may comprise any suitable method steps to determine the gut maturation status of the infant. For example, any of the method steps described in the section above entitled "Method for determining the gut maturation status of an infant".

### EXAMPLES

The invention will now be further described by way of examples, which are meant to serve to assist the skilled person in carrying out the invention and are not intended in any way to limit the scope of the invention.

### Example 1 - Consumption of products comprising an HMO mixture helps infants converge and remain convergent to a reference gut microbiome trajectory

### Materials and methods

To assess the effect of human milk oligosaccharides (HMOs) in formula feeding, a randomized controlled trial (ClinicalTrials.gov Identifier: NCT03722550) was performed. A study overview is provided in **Figure 1**.

Healthy full-term infants (7-21 days old) were randomly assigned to a standard cow's milk-based starter infant formula (control group, CG, n=154); the same formula with 1.5 g/L HMOs (test group 1, TG1, n=155); or with 2.5 g/L HMOs (test group 2, TG2, n=153); or a human milk-fed group (reference, HMG, n=61).

The standard starter infant was a bovine milk-based whey predominant term infant formula with 67 kcal/100 mL reconstituted formula, consisting of 1.9 g intact protein (70% whey/30% casein)/100 kcal, 11.1 g carbohydrates/100 kcal, and 5.3 g lipids/100 kcal. The concentration of individual HMOs in TG1 and TG2 starter infant formula is shown in **Table 1** below.

**Table 1 - concentration of individual HMOs in TG1 and TG2 starter infant formula**

| **HMO** | **Proportion (%)** |
|---|---|
| 2'-fucosyllactose (2'FL) | 58% |
| 2',3-difucosyllactose (diFL) | 6% |
| lacto-N-tetraose (LNT) | 19% |
| 3'-sialyllactose (3'-SL) | 7% |
| 6'-sialyllactose (6'-SL) | 10% |

The standard follow-up formula was a bovine milk-based whey predominant term infant formula with 67 kcal/100 mL reconstituted formula, consisting of 2 g intact protein (50% whey/50% casein)/100 kcal, 12.4 g carbohydrates/100 kcal, and 4.7 g lipids/100 kcal. The concentration of total HMOs in TG1 and TG2 follow-up formula was 0.5 g/L of the same blend as the starter infant formula.

The standard growing-up milk was a bovine milk-based growing-up milk with 67 kcal/100 mL reconstituted formula, consisting of 2.25 g intact protein (40% whey/60% casein)/100 kcal, 12.6 g carbohydrates/100 kcal, and 4.5 g lipids/100 kcal. The concentration of total HMOs in TG1 and TG2 growing-up milk was 0.4 g/L of the same blend as the starter infant formula.

Fecal samples collected at enrolment, 3, 6, 12, and 15 months of age were used for microbiome profiling. Microbial DNA was extracted from frozen faeces, purified, and shotgun sequenced with 2 x 150 bp sequencing. Taxonomic relative abundances were calculated using the metagenomic species (MGS) approach, which enables the quantification of both known characterized and uncharacterized microbial species.

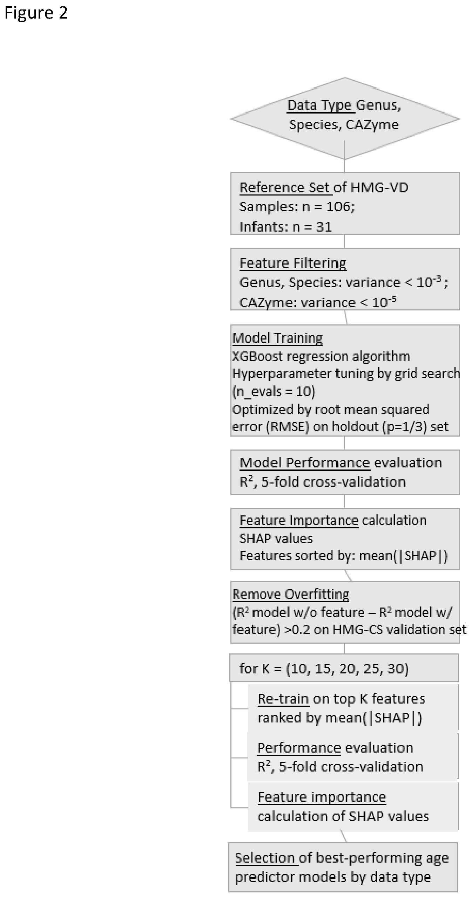

Microbiome-age predictors were trained on data from vaginally-delivered HMG infants (reference set: HMG-VD, n = 31) using Genus-level data, metagenomics Species-level (MGS) data, or CAZyme composition data and optimized with RSME (as illustrated in **Figure 2**). These models were applied to CG, TG1, and TG2 to predict microbiome-age and identify outliers (Microbiome-for-age Z-score: |MAZ|>3). Microbiome-age trajectories for CG, TG1 and TG2 were compared against the HMG-VD reference trajectory.

### Results

Selected 10 features in Genus-based model were *Romboutsia*, *Blautia*, *Staphylococcus*, *Intestinibacter*, *Cutibacterium*, *Megasphaera*, *Enterococcus*, *Bifidobacterium*, *Flavonifractor* and *Roseburia.*

Selected 20 features in Species-based model were *Romboutsia timonensis*, *Intestinibacter bartlettii*, *Staphylococcus hominis subsp. hominis*, *Staphylococcus epidermidis*, *Veillonella parvula*, [*Clostridium*] *spiroforme*, *Enterococcus faecalis*, [*Ruminococcus*] *gnavus*, *Flavonifractor plautii*, *Parabacteroides distasonis*, *Megasphaera micronuciformis*, *Bifidobacterium longum subsp. infantis*, *Bifidobacterium breve*, *Collinsella aerofaciens*, *Ruminococcaceae bacterium*, *Haemophilus parainfluenzae*, *Veillonella sp.*, *Clostridiaceae bacterium*, *Fusicatenibacter saccharivorans* and *Clostridium perfringens*.

Selected 25 features in another Species-based model were *Romboutsia timonensis*, *Intestinibacter bartlettii*, *Staphylococcus hominis subsp. hominis*, *Staphylococcus epidermidis*, *Veillonella parvula*, [*Clostridium*] *spiroforme*, *Flavonifractor plautii*, *Enterococcus faecalis*, [*Ruminococcus*] *gnavus*, *Parabacteroides distasonis*, *Megasphaera micronuciformis*, *Bifidobacterium bifidum*, *Bifidobacterium longum subsp. infantis*, *Bifidobacterium longum subsp. longum*, *Bifidobacterium breve*, *Bacteroides dorei*, *Collinsella aerofaciens*, *Ruminococcaceae bacterium*, *Veillonella sp.*, *Clostridiaceae bacterium*, *Clostridium perfringens*, *Erysipelatoclostridium ramosum*, *Fusicatenibacter saccharivorans*, *Haemophilus parainfluenzae*, *Peptostreptococcaceae sp.*

Selected 30 features in CAZymes-based model were GH13_9, GH39, GH73, GT5, CE2, CBM34, GH43_26, GT51, CBM32, GH105, GH38, GH95, GH112, GH33, GH26, GH18, GH109, CBM41, GH43_34, GH31, GH13_31, GH146, GH25, CBM48, GH43_24, GH170, GH5, CE11, GH51, GH76.

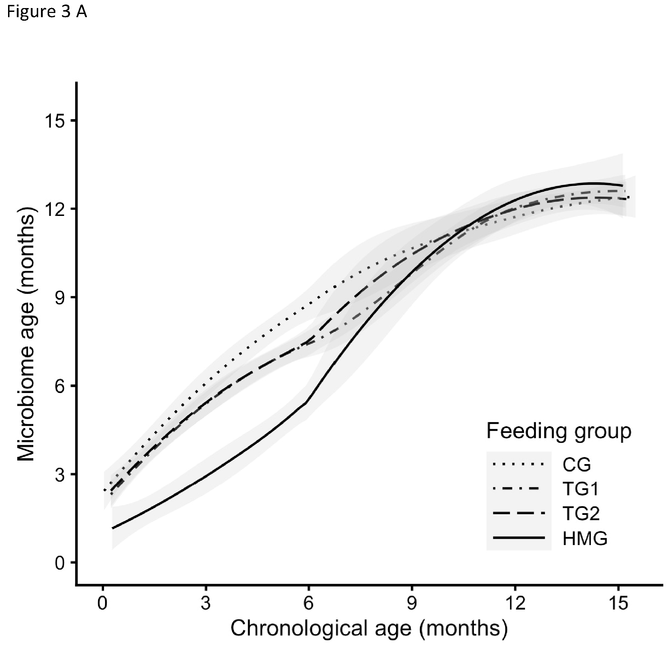

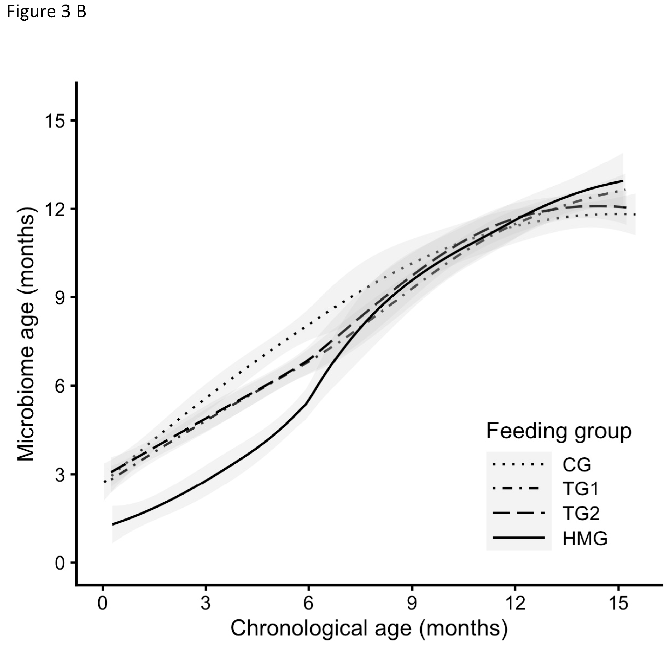

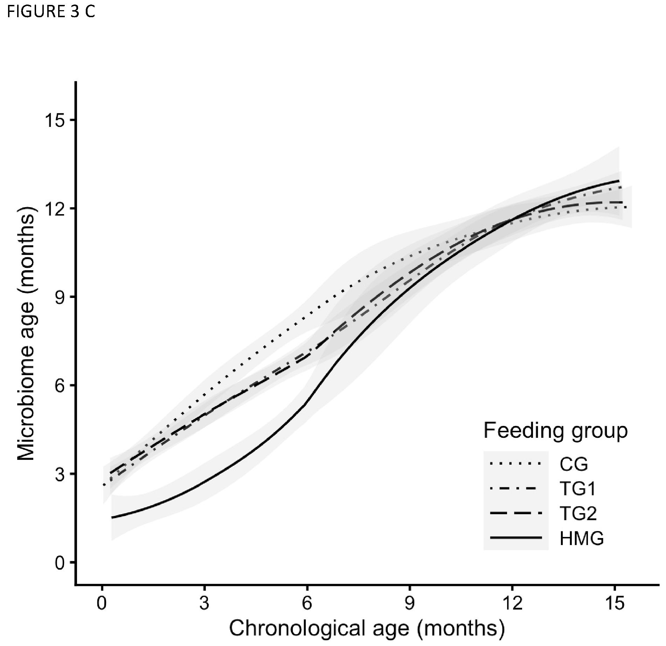

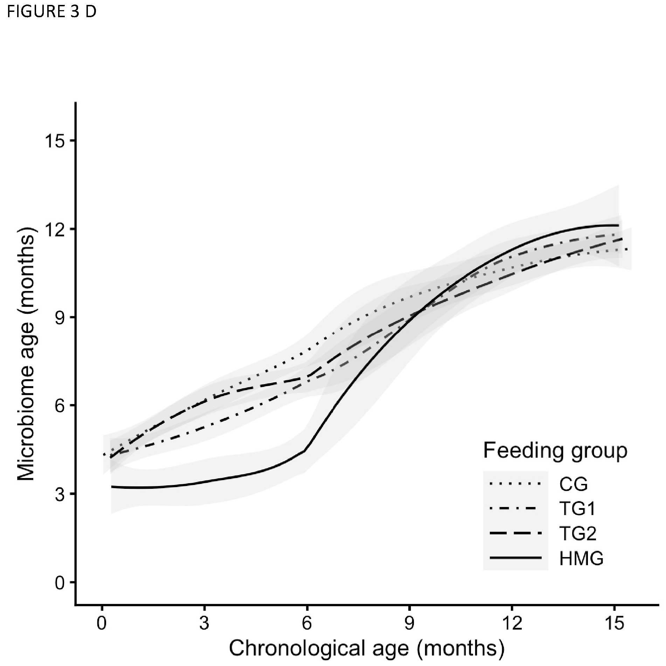

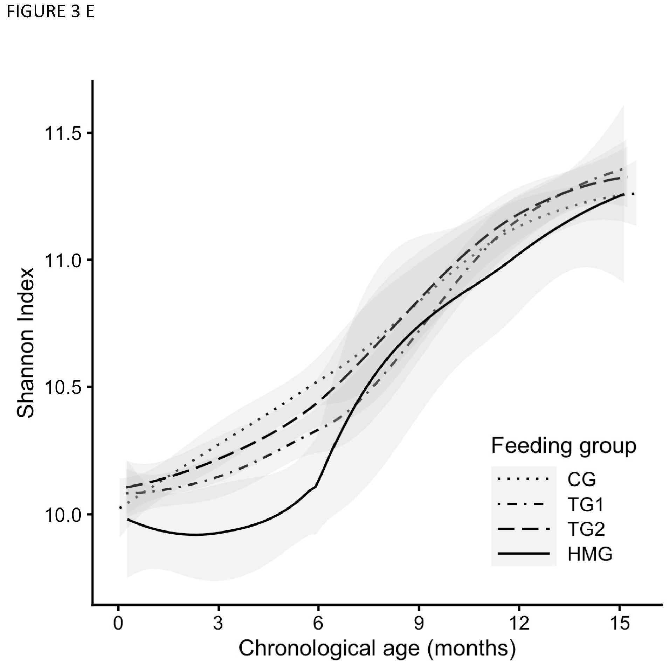

In each model, TG trajectories converged on the reference trajectory earlier than CG. For example, using a Genus-based model (with 10 features, R² = 0.862), trajectories were significantly distinct until ~11.4 months for CG, ~9.4 months for TG1, ~9.6 months for TG2 (see **Figure 3A**). Using a MGS Species-based model (with 20 features, R² = 0.881), trajectories were significantly distinct until ~10.3 months for CG, ~8.1 months for TG1, ~5.6 months for TG2 (see **Figure 3B**). Similar results were seen using a different MGS Species-based model (see **Figure 3C**), a CAZymes-based approach (see **Figure 3D**) and alpha-diversity based approach (see **Figures 3E**). This effect was more pronounced in formula-fed infants who were delivered by caesarean section (CS), as seen by different trajectories (e.g., see **Figures 4A** **and** **4B**).

After interventions started, outliers in the genus-based model were significantly reduced in TGs compared to CG using Cochran-Armitage trend test (p = 0.0002) and at visits (3-6 months p = 0.0002; 12-15 months p = 0.0377) (see **Figure 5** and **Table 2** below). Models trained on other data types indicated similar trends.

**Table 2 - Genus-based model (with selected 10 features) number of infant as outliers**

| **Group** | **No. infants with >+3 MAZ score** | **No. infants with <-3 MAZ score)** | **No. infants with >+3 and <-3 MAZ score** |
|---|---|---|---|
| CG | 64 | 23 | 14 |
| TG1 | 46 | 15 | 5 |
| TG2 | 50 | 19 | 5 |
| HMG | 5 | 3 | 1 |

These data show that the supplementation of infant formula with a mixture of HMOs can induce the gut microbiome trajectory of formula-fed infants to converge with that of human milk-fed, vaginally delivered reference infants.

### EMBODIMENTS

Various preferred features and embodiments of the present invention will now be described with reference to the following numbered paragraphs (paras).
1. A mixture of human milk oligosaccharides (HMOs) for use in inducing a formula-fed infant's gut microbiome trajectory to converge with that of a reference gut microbiome trajectory obtained from human milk-fed infants.
2. The mixture of HMOs for use according to any preceding para, wherein the mixture of HMOs comprises at least one fucosylated oligosaccharide, at least one N-acetylated oligosaccharide, and at least one sialylated oligosaccharide.
3. The mixture of HMOs for use according to any preceding para, wherein the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL), optionally wherein the mixture of HMOs comprises or consists of: (i) 2'FL in an amount of from about 55 wt% to about 60 wt%; (ii) diFL in an amount of from about 5 wt% to about 7 wt%; (iii) LNT in an amount of from about 18 wt% to about 20 wt%; (iv) 3'-SL in an amount of from about 6 wt% to about 8 wt%; and (v) 6'-SL in an amount of from about 9 wt% to about 11 wt%, based on the total weight of HMOs.
4. The mixture of HMOs for use according to any preceding para, wherein the mixture of HMOs is administered in the form of an infant formula, optionally wherein the mixture of HMOs is administered in the form of a starter infant formula, a follow-up formula, and/or a growing-up milk.
5. The mixture of HMOs for use according to any preceding para, wherein the mixture of HMOs is administered in the form of a starter infant formula comprising a total amount of HMOs of from about 0.5 g/L to about 5.0 g/L, from about 1.0 g/L to about 3.0 g/L, or from about 1.5 g/L to about 2.5 g/L; a follow-up formula comprising a total amount of HMOs of from about 0.1 g/L to about 2.0 g/L, from about 0.2 g/L to about 1.0 g/L, from about 0.3 g/L to about 0.8 g/L, from about 0.35 g/L to about 0.65 g/L; and/or a growing-up milk comprising a total amount of HMOs of from about 0.1 g/L to about 1.0 g/L, from about 0.2 g/L to about 0.8 g/L, from about 0.28 g/L to about 0.52 g/L, or from about 0.3 g/L to about 0.5 g/L.
6. The mixture of HMOs for use according to any preceding para, wherein the mixture of HMOs is administered in the form of:
   (a) a starter infant formula comprising:
      (i) 2'FL in an amount of from about 0.5 g/L to about 3.0 g/L, preferably from about 0.70 g/L to about 1.05 g/L or from about 1.16 g/L to about 1.74 g/L;
      (ii) diFL in an amount of from about 0.05 g/L to about 0.3 g/L, preferably from about 0.05 g/L to about 0.11 g/L or from about 0.12 g/L to about 0.18 g/L;
      (iii) LNT in an amount of from about 0.1 g/L to about 1.0 g/L, preferably from about 0.23 g/L to about 0.36 g/L or from about 0.39 g/L to about 0.58 g/L;
      (iv) 3'-SL in an amount of from about 0.05 g/L to about 0.3 g/L, preferably from about 0.09 g/L to about 0.13 g/L or from about 0.14 g/L to about 0.21 g/L; and
      (v) 6'-SL in an amount of from about 0.05 g/L to about 0.5 g/L, preferably from about 0.12 g/L to about 0.17 g/L or from about 0.19 g/L to about 0.28 g/L;
   (b) a follow-up formula comprising:
      (i) 2'FL in an amount of from about 0.19 g/L to about 0.34 g/L;
      (ii) diFL in an amount of from about 0.03 g/L to about 0.05 g/L;
      (iii) LNT in an amount of from about 0.06 g/L to about 0.11 g/L;
      (iv) 3'-SL in an amount of from about 0.04 g/L to about 0.09 g/L; and
      (v) 6'-SL in an amount of from about 0.03 g/L to about 0.06 g/L; and/or
   (c) growing-up milk comprising:
      (i) 2'FL in an amount of from about 0.15 g/L to about 0.28 g/L;
      (ii) diFL in an amount of from about 0.01 g/L to about 0.04 g/L;
      (iii) LNT in an amount of from about 0.05 g/L to about 0.09 g/L;
      (iv) 3'-SL in an amount of from about 0.03 g/L to about 0.08 g/L; and
      (v) 6'-SL in an amount of from about 0.03 g/L to about 0.05 g/L.
7. The mixture of HMOs for use according to any preceding para, wherein the mixture of HMOs is administered in the form of a starter infant formula comprising a total amount of HMOs of about 1.5 g/L, optionally wherein the mixture of HMOs is administered in the form of a starter infant formula comprising:
   (i) 2'FL in an amount of about 0.87 g/L;
   (ii) diFL in an amount of about 0.10 g/L;
   (iii) LNT in an amount of about 0.29 g/L;
   (iv) 3'-SL in an amount of about 0.11 g/L; and
   (v) 6'-SL in an amount of about 0.14 g/L.
8. The mixture of HMOs for use according to any of paras 1 to 6, wherein the mixture of HMOs is administered in the form of a starter infant formula comprising a total amount of HMOs of about 2.5 g/L, optionally wherein the mixture of HMOs is administered in the form of a starter infant formula comprising:
   (i) 2'FL in an amount of about 1.45 g/L;
   (ii) diFL in an amount of about 0.14 g/L;
   (iii) LNT in an amount of about 0.48 g/L;
   (iv) 3'-SL in an amount of about 0.18 g/L; and
   (v) 6'-SL in an amount of about 0.24 g/L.
9. The mixture of HMOs for use according to any preceding para, wherein the mixture of HMOs is administered in the form of a follow-up formula comprising a total amount of HMOs of about 0.5 g/L, optionally wherein the mixture of HMOs is administered in the form of a follow-up formula comprising:
   (i) 2'FL in an amount of about 0.26 g/L;
   (ii) diFL in an amount of about 0.04 g/L;
   (iii) LNT in an amount of about 0.09 g/L;
   (iv) 3'-SL in an amount of about 0.06 g/L; and
   (v) 6'-SL in an amount of about 0.05 g/L.
10. The mixture of HMOs for use according to any preceding para, wherein the mixture of HMOs is administered in the form of a growing-up milk comprising a total amount of HMOs of about 0.4 g/L, optionally wherein the mixture of HMOs is administered in the form of a growing-up milk comprising:
   (i) 2'FL in an amount of about 0.21 g/L;
   (ii) diFL in an amount of about 0.03 g/L;
   (iii) LNT in an amount of about 0.07 g/L;
   (iv) 3'-SL in an amount of about 0.06 g/L; and
   (v) 6'-SL in an amount of about 0.04 g/L.
11. The mixture of HMOs for use according to any preceding para, wherein the mixture of HMOs is administered in the form of a starter infant formula, follow-up formula, and/or growing-up milk each comprising from about 60 kcal/100mL to about 80 kcal/100mL, protein in an amount of from about 1.5 g/100kcal to about 2.5 g/100kcal, carbohydrate in an amount of from about 8 g/100kcal to about 15 g/100kcal, and lipids in an amount of from about 3 g/100kcal to about 8 g/100kcal.
12. The mixture of HMOs for use according to any preceding para, wherein the mixture of HMOs is administered to the formula-fed infant until at least about 6 months of age, until at least about 9 months of age, until at least about 12 months of age, or until at least about 15 months of age.
13. The mixture of HMOs for use according to any preceding para, wherein the formula-fed infant's gut microbiome trajectory converges with that of a reference gut microbiome trajectory obtained from human milk-fed infants at about 6 months of age or later, at about 7 months of age or later, at about 8 months of age or later, or at about 9 months of age or later.
14. The mixture of HMOs for use according to any preceding para, wherein the formula-fed infant's gut microbiome trajectory converges with that of a reference gut microbiome trajectory obtained from human milk-fed infants at about 12 months of age or earlier, about 11 months of age or earlier, about 10 months of age or earlier, or about 9 months of age or earlier.
15. The mixture of HMOs for use according to any preceding para, wherein the formula-fed infant's gut microbiome trajectory converges with that of a reference gut microbiome trajectory obtained from human milk-fed infants at about 6 to about 12 months of age, at about 7 to about 11 months of age, at about 8 to 10 months of age, or at about 9 months of age.
16. The mixture of HMOs for use according to any preceding para, wherein the gut microbiome trajectory is a gut microbiome age trajectory or a gut microbiome diversity trajectory.
17. The mixture of HMOs for use according to any preceding para, wherein the gut microbiome trajectory is a gut microbiome age trajectory, optionally wherein the gut microbiome age trajectory is obtained using genus-level data, species-level data, and/or functional data from gut microbiome data.
18. The mixture of HMOs for use according to any preceding para, wherein the formula-fed infant is full-term.
19. The mixture of HMOs for use according to any preceding para, wherein the formula-fed infant was delivered by caesarean section.
20. The mixture of HMOs for use according to any preceding para, wherein the human milk-fed infants are full-term vaginally-delivered human milk-fed infants.
21. The mixture of HMOs for use according to any preceding para, wherein inducing the formula-fed infant's gut microbiome trajectory to converge with that of a reference gut microbiome trajectory obtained from human milk-fed infants promotes gut maturation.
22. The mixture of HMOs for use according to any preceding para, wherein inducing the formula-fed infant's gut microbiome trajectory to converge with that of a reference gut microbiome trajectory obtained from human milk-fed infants promotes maturation of the gut microbiome, gut metabolism, gut barrier function and/or gut immune function.
23. A method for inducing a formula-fed infant's gut microbiome trajectory to converge with that of a reference gut microbiome trajectory obtained from human milk-fed infants, the method comprising administering an effective amount of a mixture of human milk oligosaccharides (HMOs) to the formula-fed infant.
24. Use of a mixture of human milk oligosaccharides (HMOs) to induce a formula-fed infant's gut microbiome trajectory to converge with that of a reference gut microbiome trajectory obtained from human milk-fed infants.
25. The method according to para 23 or the use according to para 24, wherein the mixture of HMOs comprises at least one fucosylated oligosaccharide, at least one N-acetylated oligosaccharide, and at least one sialylated oligosaccharide.
26. The method according to para 23 or 25 or the use according to para 24 or 25, wherein the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL), optionally wherein the mixture of HMOs comprises or consists of: (i) 2'FL in an amount of from about 55 wt% to about 60 wt%; (ii) diFL in an amount of from about 5 wt% to about 7 wt%; (iii) LNT in an amount of from about 18 wt% to about 20 wt%; (iv) 3'-SL in an amount of from about 6 wt% to about 8 wt%; and (v) 6'-SL in an amount of from about 9 wt% to about 11 wt%, based on the total weight of HMOs.
27. The method according to any of paras 23, 25 or 26 or the use according to any of paras 24 to 26, wherein the mixture of HMOs is administered in the form of an infant formula, optionally wherein the mixture of HMOs is administered in the form of a starter infant formula, a follow-up formula, and/or a growing-up milk.
28. The method according to any of paras 23 or 25 to 27 or the use according to any of paras 24 to 27, wherein the mixture of HMOs is administered in the form of a starter infant formula comprising a total amount of HMOs of from about 0.5 g/L to about 5.0 g/L, from about 1.0 g/L to about 3.0 g/L, or from about 1.5 g/L to about 2.5 g/L; a follow-up formula comprising a total amount of HMOs of from about 0.1 g/L to about 2.0 g/L, from about 0.2 g/L to about 1.0 g/L, from about 0.3 g/L to about 0.8 g/L, from about 0.35 g/L to about 0.65 g/L; and/or a growing-up milk comprising a total amount of HMOs of from about 0.1 g/L to about 1.0 g/L, from about 0.2 g/L to about 0.8 g/L, from about 0.28 g/L to about 0.52 g/L, or from about 0.3 g/L to about 0.5 g/L.
29. The method according to any of paras 23 or 25 to 28 or the use according to any of paras 24 to 28, wherein the mixture of HMOs is administered in the form of:
   (a) a starter infant formula comprising:
      (i) 2'FL in an amount of from about 0.5 g/L to about 3.0 g/L, preferably from about 0.70 g/L to about 1.05 g/L or from about 1.16 g/L to about 1.74 g/L;
      (ii) diFL in an amount of from about 0.05 g/L to about 0.3 g/L, preferably from about 0.05 g/L to about 0.11 g/L or from about 0.12 g/L to about 0.18 g/L;
      (iii) LNT in an amount of from about 0.1 g/L to about 1.0 g/L, preferably from about 0.23 g/L to about 0.36 g/L or from about 0.39 g/L to about 0.58 g/L;
      (iv) 3'-SL in an amount of from about 0.05 g/L to about 0.3 g/L, preferably from about 0.09 g/L to about 0.13 g/L or from about 0.14 g/L to about 0.21 g/L; and
      (v) 6'-SL in an amount of from about 0.05 g/L to about 0.5 g/L, preferably from about 0.12 g/L to about 0.17 g/L or from about 0.19 g/L to about 0.28 g/L;
   (b) a follow-up formula comprising:
      (i) 2'FL in an amount of from about 0.19 g/L to about 0.34 g/L;
      (ii) diFL in an amount of from about 0.03 g/L to about 0.05 g/L;
      (iii) LNT in an amount of from about 0.06 g/L to about 0.11 g/L;
      (iv) 3'-SL in an amount of from about 0.04 g/L to about 0.09 g/L; and
      (v) 6'-SL in an amount of from about 0.03 g/L to about 0.06 g/L; and/or
   (c) growing-up milk comprising:
      (i) 2'FL in an amount of from about 0.15 g/L to about 0.28 g/L;
      (ii) diFL in an amount of from about 0.01 g/L to about 0.04 g/L;
      (iii) LNT in an amount of from about 0.05 g/L to about 0.09 g/L;
      (iv) 3'-SL in an amount of from about 0.03 g/L to about 0.08 g/L; and
      (v) 6'-SL in an amount of from about 0.03 g/L to about 0.05 g/L.
30. The method according to any of paras 23 or 25 to 29 or the use according to any of paras 24 to 29, wherein the mixture of HMOs is administered in the form of a starter infant formula comprising a total amount of HMOs of about 1.5 g/L, optionally wherein the mixture of HMOs is administered in the form of a starter infant formula comprising:
   (i) 2'FL in an amount of about 0.87 g/L;
   (ii) diFL in an amount of about 0.10 g/L;
   (iii) LNT in an amount of about 0.29 g/L;
   (iv) 3'-SL in an amount of about 0.11 g/L; and
   (v) 6'-SL in an amount of about 0.14 g/L.
31. The method according to any of paras 23 or 25 to 30 or the use according to any of paras 24 to 30, wherein the mixture of HMOs is administered in the form of a starter infant formula comprising a total amount of HMOs of about 2.5 g/L, optionally wherein the mixture of HMOs is administered in the form of a starter infant formula comprising:
   (i) 2'FL in an amount of about 1.45 g/L;
   (ii) diFL in an amount of about 0.14 g/L;
   (iii) LNT in an amount of about 0.48 g/L;
   (iv) 3'-SL in an amount of about 0.18 g/L; and
   (v) 6'-SL in an amount of about 0.24 g/L.
32. The method according to any of paras 23 or 25 to 31 or the use according to any of paras 24 to 31, wherein the mixture of HMOs is administered in the form of a follow-up formula comprising a total amount of HMOs of about 0.5 g/L, optionally wherein the mixture of HMOs is administered in the form of a follow-up formula comprising:
   (i) 2'FL in an amount of about 0.26 g/L;
   (ii) diFL in an amount of about 0.04 g/L;
   (iii) LNT in an amount of about 0.09 g/L;
   (iv) 3'-SL in an amount of about 0.06 g/L; and
   (v) 6'-SL in an amount of about 0.05 g/L.
33. The method according to any of paras 23 or 25 to 32 or the use according to any of paras 24 to 32, wherein the mixture of HMOs is administered in the form of a growing-up milk comprising a total amount of HMOs of about 0.4 g/L, optionally wherein the mixture of HMOs is administered in the form of a growing-up milk comprising:
   (i) 2'FL in an amount of about 0.21 g/L;
   (ii) diFL in an amount of about 0.03 g/L;
   (iii) LNT in an amount of about 0.07 g/L;
   (iv) 3'-SL in an amount of about 0.06 g/L; and
   (v) 6'-SL in an amount of about 0.04 g/L.
34. The method according to any of paras 23 or 25 to 33 or the use according to any of paras 24 to 33, wherein the mixture of HMOs is administered in the form of a starter infant formula, follow-up formula, and/or growing-up milk each comprising from about 60 kcal/100mL to about 80 kcal/100mL, protein in an amount of from about 1.5 g/100kcal to about 2.5 g/100kcal, carbohydrate in an amount of from about 8 g/100kcal to about 15 g/100kcal, and lipids in an amount of from about 3 g/100kcal to about 8 g/100kcal.
35. The method according to any of paras 23 or 25 to 34 or the use according to any of paras 24 to 34, wherein the mixture of HMOs is administered to the formula-fed infant until at least about 6 months of age, until at least about 9 months of age, until at least about 12 months of age, or until at least about 15 months of age.
36. The method according to any of paras 23 or 25 to 35 or the use according to any of paras 24 to 35, wherein the formula-fed infant's gut microbiome trajectory converges with that of a reference gut microbiome trajectory obtained from human milk-fed infants at about 6 months of age or later, at about 7 months of age or later, at about 8 months of age or later, at about 9 months of age or later.
37. The method according to any of paras 23 or 25 to 36 or the use according to any of paras 24 to 36, wherein the formula-fed infant's gut microbiome trajectory converges with that of a reference gut microbiome trajectory obtained from human milk-fed infants at about 12 months of age or earlier, about 11 months of age or earlier, about 10 months of age or earlier, or about 9 months of age or earlier.
38. The method according to any of paras 23 or 25 to 37 or the use according to any of paras 24 to 37, wherein the formula-fed infant's gut microbiome trajectory converges with that of a reference gut microbiome trajectory obtained from human milk-fed infants at about 6 to about 12 months of age, at about 7 to about 11 months of age, at about 8 to 10 months of age, or at about 9 months of age.
39. The method according to any of paras 23 or 25 to 38 or the use according to any of paras 24 to 38, wherein the gut microbiome trajectory is a gut microbiome age trajectory or a gut microbiome diversity trajectory.
40. The method according to any of paras 23 or 25 to 39 or the use according to any of paras 24 to 39, wherein the gut microbiome trajectory is a gut microbiome age trajectory, optionally wherein the gut microbiome age trajectory is obtained using genus-level data, species-level data, and/or functional data from gut microbiome data.
41. The method according to any of paras 23 or 25 to 40 or the use according to any of paras 24 to 40, wherein the formula-fed infant is full-term.
42. The method according to any of paras 23 or 25 to 41 or the use according to any of paras 24 to 41, wherein the formula-fed infant was delivered by caesarean section.
43. The method according to any of paras 23 or 25 to 42 or the use according to any of paras 24 to 42, wherein the human milk-fed infants are full-term vaginally-delivered human milk-fed infants.
44. The method according to any of paras 23 or 25 to 43 or the use according to any of paras 24 to 43, wherein the method promotes gut maturation.
45. The method according to any of paras 23 or 25 to 44 or the use according to any of paras 24 to 44, wherein the method promotes maturation of the gut microbiome, gut metabolism, gut barrier function and/or gut immune function.
46. A method for determining the gut maturation status of a formula-fed infant, wherein the method comprises:
   (a) providing gut microbiome data from a population of human-milk fed infants;
   (b) training a regression model on the gut microbiome data; and
   (c) providing gut microbiome data from the formula-fed infant and determining whether the formula-fed infant is an outlier or not in the trained regression model,
   wherein the gut maturation status of the formula-fed infant is normal if the formula-fed infant is not an outlier in the trained regression model, and/or wherein the gut maturation status of the formula-fed infant is not normal if the formula-fed infant is an outlier in the trained regression model.
47. The method according to para 46, wherein the formula-fed infant is an outlier based on the standard errors (SE), confidence intervals, prediction intervals, and/or standard deviations in the trained regression model, preferably wherein the formula-fed infant is an outlier if their gut microbiome data is -2SE or less or 2SE or more, -2.5SE or less or 2.5SE or more, or -3SE or less or 3SE or more from the trained regression line, if their gut microbiome data falls outside the 90%, 95% or 99% confidence interval in the trained regression model, if their gut microbiome data falls outside the 90%, 95% or 99% prediction interval in the trained regression model, and/or if they have a Z-score of -2 or less or 2 or more, -2.5 or less or 2.5 or more, or -3 or less or 3 or more in the trained regression model.
48. A method for determining the gut maturation status of a formula-fed infant, wherein the method comprises:
   (a) providing gut microbiome data from a population of human-milk fed infants;
   (b) training a regression model on the gut microbiome data to provide a gut microbiome trajectory; and
   (c) providing gut microbiome data from the formula-fed infant and determining whether the formula-fed infant is on or off the gut microbiome trajectory,
   wherein the gut maturation status of the formula-fed infant is normal if the formula-fed infant is on the gut microbiome trajectory, and/or wherein the gut maturation status of the formula-fed infant is not normal if the formula-fed infant is off the gut microbiome trajectory.
49. The method according to para 48, wherein the formula-fed infant is determined to be off the gut microbiome trajectory based on the standard errors (SE), confidence intervals, prediction intervals, and/or standard deviations of the gut microbiome trajectory, preferably the formula-fed infant is determined to be off the gut microbiome trajectory if their gut microbiome data is -2SE or less or 2SE or more, -2.5SE or less or 2.5SE or more, or -3SE or less or 3SE or more from the gut microbiome trajectory, if their gut microbiome data falls outside the 90%, 95% or 99% confidence interval of the gut microbiome trajectory, if their gut microbiome data falls outside the 90%, 95% or 99% prediction interval of the gut microbiome trajectory, and/or if they have a Z-score of -2 or less or 2 or more, -2.5 or less or 2.5 or more, or -3 or less or 3 or more.
50. A data processing system comprising means for carrying out the method according to any of paras 46 to 49.
51. A data processing system comprising a processor configured to perform the method according to any of paras 46 to 49.
52. A computer-readable medium comprising instructions which, when executed by a computer, cause the computer to carry out the method according to any of paras 46 to 49.
53. A computer program comprising instructions which, when the program is executed by a computer, cause the computer to carry out the method according to any of paras 46 to 49.
54. A computer-readable data carrier having stored thereon a computer program according to para 53.
55. A data carrier signal carrying a computer program according to para 53.
56. Use of one or more reference gut microbiome trajectories obtained from human milk-fed infants to determine the gut maturation status of a formula-fed infant following administration of a mixture of HMOs.

All publications mentioned in the above specification are herein incorporated by reference. Various modifications and variations of the disclosed methods, compositions and uses of the invention will be apparent to the skilled person without departing from the scope and spirit of the invention. Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person are intended to be within the scope of the following claims.

## Claims

1. A mixture of human milk oligosaccharides (HMOs) for use in inducing a formula-fed infant's gut microbiome trajectory to converge with that of a reference gut microbiome trajectory obtained from human milk-fed infants, wherein the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL).

2. The mixture of HMOs for use according to claim 1, wherein the mixture of HMOs comprises or consists of: (i) 2'FL in an amount of from about 55 wt% to about 60 wt%; (ii) diFL in an amount of from about 5 wt% to about 7 wt%; (iii) LNT in an amount of from about 18 wt% to about 20 wt%; (iv) 3'-SL in an amount of from about 6 wt% to about 8 wt%; and (v) 6'-SL in an amount of from about 9 wt% to about 11 wt%, based on the total weight of HMOs.

3. The mixture of HMOs for use according to claim 1 or 2, wherein the mixture of HMOs is administered in the form of an infant formula, optionally wherein the mixture of HMOs is administered in the form of a starter infant formula, a follow-up formula, and/or a growing-up milk.

4. The mixture of HMOs for use according to any preceding claim, wherein the mixture of HMOs is administered in the form of:
(a) a starter infant formula comprising a total amount of HMOs of from about 0.5 g/L to about 5.0 g/L, from about 1.0 g/L to about 3.0 g/L, or from about 1.5 g/L to about 2.5 g/L, optionally wherein the starter infant formula comprises:
(i) 2'FL in an amount of from about 0.5 g/L to about 3.0 g/L, preferably from about 0.70 g/L to about 1.05 g/L or from about 1.16 g/L to about 1.74 g/L;
(ii) diFL in an amount of from about 0.05 g/L to about 0.3 g/L, preferably from about 0.05 g/L to about 0.11 g/L or from about 0.12 g/L to about 0.18 g/L;
(iii) LNT in an amount of from about 0.1 g/L to about 1.0 g/L, preferably from about 0.23 g/L to about 0.36 g/L or from about 0.39 g/L to about 0.58 g/L;
(iv) 3'-SL in an amount of from about 0.05 g/L to about 0.3 g/L, preferably from about 0.09 g/L to about 0.13 g/L or from about 0.14 g/L to about 0.21 g/L; and
(v) 6'-SL in an amount of from about 0.05 g/L to about 0.5 g/L, preferably from about 0.12 g/L to about 0.17 g/L or from about 0.19 g/L to about 0.28 g/L;
(b) a follow-up formula comprising a total amount of HMOs of from about 0.1 g/L to about 2.0 g/L, from about 0.2 g/L to about 1.0 g/L, from about 0.3 g/L to about 0.8 g/L, from about 0.35 g/L to about 0.65 g/L, optionally wherein the follow-up formula comprises:
(i) 2'FL in an amount of from about 0.19 g/L to about 0.34 g/L;
(ii) diFL in an amount of from about 0.03 g/L to about 0.05 g/L;
(iii) LNT in an amount of from about 0.06 g/L to about 0.11 g/L;
(iv) 3'-SL in an amount of from about 0.04 g/L to about 0.09 g/L; and
(v) 6'-SL in an amount of from about 0.03 g/L to about 0.06 g/L; and/or
(c) a growing-up milk comprising a total amount of HMOs of from about 0.1 g/L to about 1.0 g/L, from about 0.2 g/L to about 0.8 g/L, from about 0.28 g/L to about 0.52 g/L, or from about 0.3 g/L to about 0.5 g/L, optionally wherein the growing-up milk comprises:
(i) 2'FL in an amount of from about 0.15 g/L to about 0.28 g/L;
(ii) diFL in an amount of from about 0.01 g/L to about 0.04 g/L;
(iii) LNT in an amount of from about 0.05 g/L to about 0.09 g/L;
(iv) 3'-SL in an amount of from about 0.03 g/L to about 0.08 g/L; and
(v) 6'-SL in an amount of from about 0.03 g/L to about 0.05 g/L.

5. The mixture of HMOs for use according to any preceding claim, wherein the mixture of HMOs is administered in the form of:
(a) a starter infant formula comprising a total amount of HMOs of about 1.5 g/L or 2.5 g/L, optionally wherein the starter infant formula comprises:
(i) 2'FL in an amount of about 0.87 g/L or about 1.45 g/L;
(ii) diFL in an amount of about 0.10 g/L or about 0.14 g/L;
(iii) LNT in an amount of about 0.29 g/L or about 0.48 g/L;
(iv) 3'-SL in an amount of about 0.11 g/L or about 0.18 g/L; and
(v) 6'-SL in an amount of about 0.14 g/L or about 0.24 g/L;
(b) a follow-up formula comprising a total amount of HMOs of about 0.5 g/L, optionally wherein the follow-up formula comprises:
(i) 2'FL in an amount of about 0.26 g/L;
(ii) diFL in an amount of about 0.04 g/L;
(iii) LNT in an amount of about 0.09 g/L;
(iv) 3'-SL in an amount of about 0.06 g/L; and
(v) 6'-SL in an amount of about 0.05 g/L; and/or
(c) a growing-up milk comprising a total amount of HMOs of about 0.4 g/L, optionally wherein the growing-up milk comprises:
(i) 2'FL in an amount of about 0.21 g/L;
(ii) diFL in an amount of about 0.03 g/L;
(iii) LNT in an amount of about 0.07 g/L;
(iv) 3'-SL in an amount of about 0.06 g/L; and
(v) 6'-SL in an amount of about 0.04 g/L.

6. The mixture of HMOs for use according to any preceding claim, wherein the mixture of HMOs is administered to the formula-fed infant until at least about 6 months of age, until at least about 9 months of age, until at least about 12 months of age, or until at least about 15 months of age.

7. The mixture of HMOs for use according to any preceding claim, wherein the formula-fed infant's gut microbiome trajectory converges with that of a reference gut microbiome trajectory obtained from human milk-fed infants at about 6 to about 12 months of age, at about 7 to about 11 months of age, at about 8 to 10 months of age, or at about 9 months of age.

8. The mixture of HMOs for use according to any preceding claim, wherein inducing the formula-fed infant's gut microbiome trajectory to converge with that of a reference gut microbiome trajectory obtained from human milk-fed infants promotes gut maturation.

9. A method for inducing a formula-fed infant's gut microbiome trajectory to converge with that of a reference gut microbiome trajectory obtained from human milk-fed infants, the method comprising administering an effective amount of a mixture of human milk oligosaccharides (HMOs) to the formula-fed infant, wherein the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL).

10. Use of a mixture of human milk oligosaccharides (HMOs) to induce a formula-fed infant's gut microbiome trajectory to converge with that of a reference gut microbiome trajectory obtained from human milk-fed infants, wherein the mixture of HMOs comprises or consists of 2'-fucosyllactose (2'FL), 2',3-difucosyllactose (diFL), lacto-N-tetraose (LNT), 3'-sialyllactose (3'-SL), and 6'-sialyllactose (6'-SL).

11. A method for determining the gut maturation status of a formula-fed infant, wherein the method comprises:
(a) providing gut microbiome data from a population of human-milk fed infants;
(b) training a regression model on the gut microbiome data; and
(c) providing gut microbiome data from the formula-fed infant and determining whether the formula-fed infant is an outlier or not in the trained regression model,
wherein the gut maturation status of the formula-fed infant is normal if the formula-fed infant is not an outlier in the trained regression model, and/or wherein the gut maturation status of the formula-fed infant is not normal if the formula-fed infant is an outlier in the trained regression model.

12. A method for determining the gut maturation status of a formula-fed infant, wherein the method comprises:
(a) providing gut microbiome data from a population of human-milk fed infants;
(b) training a regression model on the gut microbiome data to provide a gut microbiome trajectory; and
(c) providing gut microbiome data from the formula-fed infant and determining whether the formula-fed infant is on or off the gut microbiome trajectory,
wherein the gut maturation status of the formula-fed infant is normal if the formula-fed infant is on the gut microbiome trajectory, and/or wherein the gut maturation status of the formula-fed infant is not normal if the formula-fed infant is off the gut microbiome trajectory.

13. A computer program comprising instructions which, when the program is executed by a computer, cause the computer to carry out the method according to claim 11 or 12.

14. A data carrier signal carrying a computer program according to claim 13.

15. Use of one or more reference gut microbiome trajectories obtained from human milk-fed infants to determine the gut maturation status of a formula-fed infant following administration of a mixture of HMOs.
